(19) Europäisches Patentamt
European Patent Office
Office européen des brevets

(11) Veröffentlichungsnummer: **0 005 559**
**B1**

(12) # EUROPÄISCHE PATENTSCHRIFT

(45) Veröffentlichungstag der Patentschrift: **04.11.81**

(51) Int. Cl.³: **C 07 D 333/36,** A 61 K 31/38

(21) Anmeldenummer: **79101594.4**

(22) Anmeldetag: **23.05.79**

(54) **Phenylaminothiophenessigsäuren, Verfahren zu ihrer Herstellung und sie enthaltende Arzneimittel.**

(30) Priorität: **24.05.78 CH 5687/78**
**10.04.79 CH 3419/79**

(43) Veröffentlichungstag der Anmeldung: **28.11.79 Patentblatt 79/24**

(45) Bekanntmachung des Hinweises auf die Patenterteilung: **04.11.81 Patentblatt 81/44**

(84) Benannte Vertragsstaaten: **AT CH DE IT LU NL SE**

(56) Entgegenhaltungen: **CH-A-534 688**

(73) Patentinhaber: **Byk Gulden Lomberg Chemische Fabrik GmbH, Byk-Gulden-Strasse 2, D-7750 Konstanz (DE)**

(72) Erfinder: **Figala, Volker, Dr., Am Hochfürst 2, D-7753 Allensbach 4 (DE)**
Erfinder: **Rainer, Georg, Dr., J.A.-Feuchtmayer.Strasse 7, D-7750 Konstanz (DE)**
Erfinder: **Riedel, Richard, Dr., Bruelstrasse 22, D-7750 Konstanz (DE)**

**EP 0 005 559 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Phenylaminothiophenessigsäuren, Verfahren zu ihrer Herstellung und sie enthaltende Arzneimittel

Die Erfindung betrifft Phenylaminothiophenessigsäuren, Verfahren zu ihrer Herstellung und sie enthaltende Arzneimittel

Die erfindungsgemäßen Verbindungen werden in der pharmazeutischen Industrie und zur Herstellung von Medikamenten verwendet.

In der DE-PS 1 493 705 werden 3-Phenylaminothiophen-4-carbonsäuren beschrieben, denen gute antiphlogistische und antipyretische Eigenschaften zugeschrieben werden. Die 2,3-Dimethylphenylverbindung erwies sich dabei dem Phenylbutazon in der antiphlogistischen Wirkung vergleichbar und in der antipyretischen Wirkung überlegen. Weitere interessante Vertreter dieser Verbindungsklasse sind das 2,6-Dichlorphenyl- und das 2-Methyl-3-chlorphenylderivat [H. G. Alpermann, Arzneim.-Forsch. (Drug Res.) 20 (1970) 293–94] und das 2-Chlor-3-methylphenylderivat [H. G. Alpermann et al., Arzneim.-Forsch. (Drug Res.) 22 (1972) 2146–47], die dem Phenylbutazon in der Wirkung überlegen sind. Verschiedenen Thiophencarbonsäuren werden außerdem fibrinolytische Eigenschaften zugeschrieben [K. N. von Kaulla, Arzneim.-Forsch. 25, 152–55 (1975); K. N. von Kaulla, D. Thilo, Klin. Wochensch. 48, 668–73 (1970); D. Thilo, K. N. von Kaulla, J. Med. Chem. 13, 503–10 (1970)].

Es wurde nun gefunden, daß Phenylaminothiophenessigsäuren (= Phenylaminothienylessigsäuren) und ihre Salze eine hervorragende pharmakologische Wirksamkeit aufweisen und daß die entsprechenden Nitrile oder Ester überwiegend wertvolle Zwischenprodukte zur Herstellung der pharmakologisch wirksamen Phenylaminothiophenessigsäuren bzw. ihrer Salze darstellen.

Gegenstand der Erfindung sind Phenylaminothiophenessigsäuren der allgemeinen Formel I

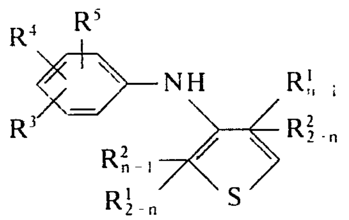

(I)

worin

$R^1$ ein Wasserstoffatom, ein Chloratom, ein Bromatom oder eine Methylgruppe,
$R^2$ eine $-CH_2-COOH$-Gruppe oder eine $-CH_2-COOR^6$-Gruppe und
$R^3$ ein Wasserstoffatom, ein Halogenatom, eine Alkylgruppe, eine Alkoxygruppe oder eine Trifluormethylgruppe bedeuten,
$R^4$ eine der Bedeutungen von $R^3$ hat,
$R^5$ ein Wasserstoffatom, ein Halogenatom oder eine Alkylgruppe,
$R^6$ eine gegebenenfalls durch Hydroxy-, Hydroxyalkoxy- oder Alkanoyloxygruppen substituierte Alkylgruppe mit 1 bis 5 Kohlenstoffatomen oder eine Benzylgruppe und
n 1 oder 2 bedeuten,

sowie die Salze der Säuren.

Als Halogenatome kommen das Fluor- oder Bromatom, bevorzugt das Chloratom in Betracht.

Als Alkyl- oder Alkoxygruppen kommen solche mit 1 bis 5 Kohlenstoffatomen in Betracht. Beispielsweise seien genannt solche mit geradkettigen Alkylgruppen, wie die Pentyl-, Butyl-, Propyl-, Ethyl- oder Methylgruppe, und solche mit verzweigten Alkylgruppen, wie die Neopentyl-, tert.-Butyl-, sek.-Butyl- oder Isopropylgruppe. Bevorzugte Alkyl- oder Alkoxygruppen sind die Methyl- und Methoxygruppe.

Als Hydroxyalkoxygruppen kommen solche mit bis zu vier Kohlenstoffatomen und bis zu drei Hydroxygruppen in Betracht. Als Alkanoyloxygruppen kommen solche mit eins bis vier Kohlenstoffatomen in Betracht. Beispielsweise seien genannt die 2-Hydroxyethoxy-, die 2,3-Dihydroxypropoxy-, die 2-Hydroxypropoxy-, die Acetoxy- und die Butyryloxygruppe. Bevorzugte substituierte Alkylgruppen $R^6$ sind die 2-Hydroxy-ethoxy-ethyl- und die Acetoxymethylgruppe.

Unter den Salzen der Erfindung sind die pharmakologisch akzeptablen, d. h. biologisch verträglichen Salze bevorzugt. Als Kationen für die Salzbildung werden vor allem die Kationen der Alkali-, Erdalkali- und Erdmetalle verwendet, oder das Ammoniumion, aber auch die korrespondierenden Kationensäuren ein- oder mehrwertiger organischer Stickstoffbasen, insbesondere organische Amine.

Beispielsweise kommen die Kationen der Metalle Lithium, Natrium, Kalium, Magnesium, Calcium und Aluminium oder deren Mischungen, wie in basischen Magnesium-Aluminium-Komplexsalzen, Kupfer und die Kationensäuren von Ethanolamin, Diethanolamin, Triethanolamin, Ethylendiamin, Dimethylamin, Diethylamin, Morpholin, Piperazin, Methylcyclohexylamin, Glucosamin, N-Methylglucamin, N-Methylglucosamin, ferner von tert.-Butylamin, Dibutylamin, Diisopropylamin, Triethylamin, Isopropylamin, 2-Amino-2-thiazolin, Chinolin, Ammoniak oder Aminosäuren, wie Alanin, Lysin, Arginin oder Asparagin, in Betracht.

Eine Ausgestaltung der Erfindung sind Phenylaminothiophenessigsäuren der allgemeinen Formel I*

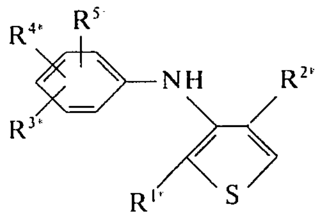

(I*)

worin

$R^{1*}$ ein Wasserstoffatom, ein Chloratom, ein Bromatom oder eine Methylgruppe,
$R^{2*}$ eine $-CH_2-COOH$-Gruppe oder eine $-CH_2-COOR^{6*}$-Gruppe und
$R^{3*}$ ein Wasserstoffatom, ein Chloratom, eine Methylgruppe oder eine Trifluormethylgruppe bedeuten,
$R^{4*}$ eine der Bedeutungen von $R^{3*}$ hat,
$R^{5*}$ ein Wasserstoffatom oder ein Chloratom und
$R^{6*}$ eine gegebenenfalls durch eine Acetoxygruppe oder eine 2-Hydroxyethoxygruppe substituierte Alkylgruppe mit 1 bis 4 Kohlenstoffatomen oder eine Benzylgruppe bedeuten,

sowie die Salze der Säuren.

Bevorzugte Vertreter der Ausgestaltung I* sind solche, in denen $R^{1*}$ ein Wasserstoffatom oder ein Chloratom, $R^{2*}$ eine $-CH_2-COOH$-Gruppe oder eine $-CH_2-COOR^{6*}$-Gruppe, $R^{3*}$ ein Chloratom, eine Methylgruppe oder eine Trifluormethylgruppe, $R^{4*}$ ein Chloratom oder eine Methylgruppe, $R^{5*}$ ein Wasserstoffatom oder ein Chloratom und $R^{6*}$ eine 2-(2-Hydroxyethoxy)-ethylgruppe bedeuten, sowie die pharmakologisch verträglichen Salze der Säuren.

Besonders bevorzugte Vertreter der Ausgestaltung I* sind solche, in denen $R^{1*}$ ein Wasserstoffatom, $R^{2*}$ eine $-CH_2-COOH$-Gruppe, $R^{3*}$ ein Chloratom oder eine Methylgruppe, $R^{4*}$ ein Chloratom und $R^{5*}$ ein Wasserstoffatom bedeuten, sowie ihre pharmakologisch verträglichen Salze.

Eine weitere Ausgestaltung der Erfindung sind Phenylaminothiophenessigsäuren der allgemeinen Formel I**

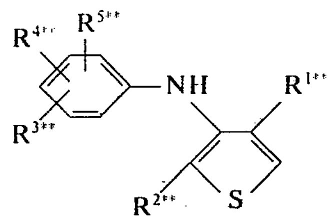

(I**)

worin

$R^{1**}$ ein Wasserstoffatom oder eine Methylgruppe,
$R^{2**}$ eine $-CH_2-COOH$-Gruppe oder eine $-CH_2-COOR^{6**}$-Gruppe und
$R^{3**}$ ein Wasserstoffatom, ein Chloratom, eine Methylgruppe oder eine Trifluormethylgruppe bedeuten,
$R^{4**}$ eine der Bedeutungen von $R^{3**}$ hat,
$R^{5**}$ ein Wasserstoffatom oder ein Chloratom und
$R^{6**}$ eine gegebenenfalls durch eine Acetoxygruppe oder eine 2-Hydroxyethoxygruppe substituierte Alkylgruppe mit 1 bis 4 Kohlenstoffatomen oder eine Benzylgruppe bedeuten,

sowie die Salze der Säuren.

Bevorzugte Vertreter der Ausgestaltung I** sind solche, in denen $R^{1**}$ ein Wasserstoffatom, $R^{2**}$ eine $-CH_2-COOH$-Gruppe oder eine $-CH_2-COOR^{6**}$-Gruppe, $R^{3**}$ ein Chloratom, eine Methylgruppe oder eine Trifluormethylgruppe, $R^{4**}$ ein Chloratom oder eine Methylgruppe, $R^{5**}$ ein Wasserstoffatom oder ein Chloratom und $R^{6**}$ eine 2-(2-Hydroxyethoxy)-ethylgruppe bedeuten, sowie die pharmakologisch verträglichen Salze der Säuren.

Besonders bevorzugte Vertreter der Ausgestaltung I** sind solche, in denen $R^{1**}$ ein Wasserstoffatom, $R^{2**}$ eine $-CH_2-COOH$-Gruppe, $R^{3**}$ ein Chloratom oder eine Methylgruppe, $R^{4**}$ ein Chloratom und $R^{5**}$ ein Wasserstoffatom bedeuten, sowie ihre pharmakologisch verträglichen Salze.

Vertreter der Formel I* sind gegenüber denen der Formel I** bevorzugt.

Vertreter der erfindungsgemäßen Verbindungen sind beispielsweise

4-(2,3-Dimethylanilino)-3-thiophenessigsäure,
4-(2,3-Dimethylanilino)-3-thiophenessigsäurebenzylester,
4-(2-Fluor-6-trifluormethylanilino)-3-thiophenessigsäure,
4-(2-Chlor-6-methylanilino)-3-thiophenessigsäure,
4-(2-Chlor-6-methylanilino)-3-thiophenessigsäure-2-(2-hydroxyethoxy)-ethylester,
4-(2-Chlor-6-methylanilino)-3-thiophenessigsäurebenzylester,
4-(2,4,6-Trimethylanilino)-3-thiophenessigsäure,
4-(2,4,6-Trichloranilino)-3-thiophenessigsäure,
4-(2,4,6-Trimethylanilino)-3-thiophenessigsäure-3-acetoxy-propylester,
4-(2-Bromanilino)-3-thiophenessigsäure,
4-(2-Methyl-4-trifluormethylanilino)-3-thiophenessigsäure,
4-(4-Fluoranilino)-3-thiophenessigsäure,
4-(2,3-Dichloranilino)-3-thiophenessigsäure,
4-(2,3-Dichloranilino)-3-thiophenessigsäuremethylester,
4-(2,3-Dichloranilino)-3-thiophenessigsäure-2-(2-hydroxyethoxy)-ethylester,
4-(2,3-Dichloranilino)-3-thiophenessigsäurebenzylester,
4-(2-Chlor-5-methylanilino)-3-thiophenessigsäure,
4-(2-Chlor-5-methylanilino)-3-thiophenessigsäure-2-acetoxy-methylester,
4-(2-Chlor-3-methylanilino)-3-thiophenessigsäure-3-acetoxy-propylester,
4-(2-Chlor-3-methylanilino)-3-thiophenessigsäurebenzylester,
4-(2,4-Dichlor-3-methylanilino)-3-thiophenessigsäure,
4-(2,4-Dichlor-3-methylanilino)-3-thiophenessigsäure-n-propylester,
4-(2,4-Dichlor-5-methylanilino)-3-thiophenessigsäure,
4-(2,4-Dichlor-5-methylanilino)-3-thiophenessigsäure-sec.-butylester,
3-(2,3-Dimethylanilino)-2-thiophenessigsäure,
3-(2,3-Dimethylanilino)-2-thiophenessigsäure-2-(2-hydroxyethoxy)-ethylester,
3-(2,4,6-Trimethylanilino)-2-thiophenessigsäure,
3-(2,4,6-Trichloranilino)-2-thiophenessigsäure,
3-(2-Chloranilino)-2-thiophenessigsäure,
3-(2-Chloranilino)-2-thiophenessigsäure-3-acetoxy-propylester,
3-(2-Chloranilino)-2-thiophenessigsäurebenzylester,
3-(2,3-Dichloranilino)-2-thiophenessigsäure,
3-(2,3-Dichloranilino)-2-thiophenessigsäure-2-butyryloxy-ethylester,
3-(2,3-Dichloranilino)-2-thiophenessigsäurebenzylester,
3-(3-Methylanilino)-2-thiophenessigsäure,
3-(3-Methylanilino)-2-thiophenessigsäureisopropylester,
3-(3-Methylanilino)-2-thiophenessigsäurebenzylester,
3-(2,4-Dichlor-3-methylanilino)-2-thiophenessigsäure,
3-(2,4-Dichlor-3-methylanilino)-2-thiophenessigsäure-2-(2-hydroxyethoxy)-ethylester,
3-(2,4-Dichlor-3-methylanilino)-2-thiophenessigsäurebenzylester,
4-(2-Methyl-4-trifluormethylanilino)-3-thiophenessigsäure,
3-(2-Methyl-4-trifluormethylanilino)-2-thiophenessigsäure-n-butylester,
3-(2-Methyl-4-trifluormethylanilino)-2-thiophenessigsäure-2-acetoxy-methylester,
3-(2,4-Dichloranilino)-2-thiophenessigsäure-benzylester,
3-(2,5-Dichloranilino)-2-thiophenessigsäure,
4-(2,5-Dichloranilino)-3-thiophenessigsäurebenzylester,
4-(2,3-Dimethylanilino)-5-chlor-3-thiophenessigsäure,
4-(2,3-Dimethylanilino)-5-chlor-3-thiophenessigsäure-2-(2-hydroxyethoxy)-ethylester,
4-(2-Chlor-6-methylanilino)-5-chlor-3-thiophenessigsäure,
4-(2-Bromanilino)-5-chlor-3-thiophenessigsäure,
4-(2,3-Dichloranilino)-5-methyl-3-thiophenessigsäure,
4-(2,3-Dichloranilino)-5-methyl-3-thiophenessigsäure-bentylester,
4-(2-Chlor-5-methylanilino)-5-methyl-3-thiophenessigsäure,
4-(2,4-Dichlor-3-methylanilino)-5-chlor-3-thiophenessigsäure,
4-Methyl-3-(2,3-dimethylanilino)-2-thiophenessigsäure,
4-Methyl-3-(2,3-dimethylanilino)-2-thiophenessigsäure-2-acetoxy-methylester,
3-(2-Chloranilino)-4-methyl-2-thiophenessigsäure,
3-(2-Chloranilino)-4-methyl-2-thiophenessigsäurebenzylester,
3-(2,3-Dichloranilino)-4-methyl-2-thiophenessigsäure,
5-Chlor-4-(2-methyl-4-trifluormethylanilino)-3-thiophenessigsäure,
4-(2,4,6-Trichloranilino)-5-methyl-3-thiophenessigsäure,
5-Chlor-4-(2,4,6-trichloranilino)-3 thiophenessigsäure,
sowie die Salze der Säuren.

Bevorzugte Verbindungen sind:

4-(2,6-Dichloranilino)-3-thiophenessigsäure,
4-(2-Chlor-6-methylanilino)-3-thiophenessigsäure,
4-(2,6-Dichlor-3-methylanilino)-3-thiophenessigsäure,
3-(2,6-Dichloranilino)-2-thiophenessigsäure,
4-(2-Chlor-3-methylanilino)-3-thiophenessigsäure,
4-(3-Chlor-2-methylanilino)-3-thiophenessigsäure,
4-(2,6-Dimethylanilino)-3-thiophenessigsäure,
5-Chlor-4-(2,6-dichloranilino)-3-thiophenessigsäure,
3-(2-Chlor-6-methylanilino)-2-thiophenessigsäure und deren Salze.

Die erfindungsgemäßen Verbindungen und ihre Salze weisen ausgeprägte antiphlogistische neben analgetischen und antipyretischen Eigenschaften auf, wie sich z. B. durch verschiedene Tests, in denen der Einfluß der Verbindungen auf akute Entzündungsreaktionen (Carrageenin-Ödem der Rattenhinterpfote [Winter et al.: Proc. Soc. exp. Biol. Med. 111 (1962) 544]) sowie chronische Entzündungsvorgänge (Cotton-pellet-Test an der Ratte [Winter et al.: J. Pharmacol. exp. Therap. 141 (1963) 369)]) und die Adjuvans-Arthritis [in Anlehnung an Perrine et al., Brit. J. Pharmacol. 21 (1963) 127] bestimmt wird, nachweisen läßt. Dabei erweisen sie sich den Verbindungen des Standes der Technik, z. B. denen der genannten DE-PS 1 493 705 bzw. den handelsüblichen Arzneimitteln, wie Indometacin, überlegen. Darüber hinaus zeichnen sie sich durch eine vergleichsweise geringe Toxizität aus.

Bei Applikation einer therapeutisch wirksamen und pharmakologisch verträglichen Menge eignen sich die erfindungsgemäßen Verbindungen daher für die Behandlung einer Vielzahl von Säugetierkrankheitszuständen, bei denen ein oder mehrere Symptome von Entzündungen, Schmerzen und Fieber auftreten. Beispiele derartiger Krankheitszustände sind die verschiedensten entzündlichen und degenerativen Erkrankungen des rheumatischen Formenkreises und anderer entzündlicher Krankheitsprozesse, z. B. akute und chronische Polyarthritis, Osteoarthritis, psoriatische Arthritis, Ankylosespondylitis, Polyarthrosen, Spondylosen, Gelenkrheumatismus, rheumatisches Fieber; Weichteilrheumatismus, z. B. Ischias; schmerzhafte postoperative Schwellungen und Entzündungen; Schmerzen und Schwellungen nach Gelenkergüssen, Verstauchungen und Brüchen; Schmerzen und Entzündungen im Zusammenhang mit der Zahnchirurgie; Schmerzzustände der verschiedensten Genese, z. B. Neuritiden, Kopfschmerzen und Spasmen; sowie menschliche und tierische Krankheitszustände, welche die vorstehenden Symptome ergeben und die Anwendung eines entzündungshemmenden, analgetischen und/oder antipyretischen Arzneimittels erfordern.

Die erfindungsgemäßen Verbindungen werden daher zur Behandlung von Säugetieren, die mit einem oder mehreren Symptomen von Entzündungen, Schmerzen oder Fieber erkrankt sind, eingesetzt, wobei man dem erkrankten Säugetier eine therapeutisch wirksame und pharmakologisch verträgliche Menge einer oder mehrerer Verbindungen der allgemeinen Formeln I, I* oder I** und/oder deren Salze verabreicht. Gegenstand der Erfindung sind somit auch die erfindungsgemäßen Verbindungen zur Verwendung bei der Bekämpfung der oben angegebenen Krankheiten. Ebenso umfaßt die Erfindung die Verwendung der erfindungsgemäßen Verbindungen zur Herstellung von Arzneimitteln, die zur Bekämpfung der angeführten Krankheiten eingesetzt werden.

Gegenstand der Erfindung sind ferner Arzneimittel, die durch den Gehalt an einem oder mehreren der neuen Wirkstoffe gekennzeichnet sind. Gegebenenfalls enthalten die neuen Arzneimittel neben den neuen Wirkstoffen pharmazeutische Trägerstoffe für diese Wirkstoffe. Der Wirkstoffgehalt dieser Arzneimittel beträgt 1 bis 95, vorzugsweise 10 bis 85 Gewichtsprozent, bezogen auf das fertige Arzneimittel.

Die Arzneimittel werden nach an sich bekannten Verfahren hergestellt. Als Arzneimittel werden die erfindungsgemäßen Verbindungen entweder als solche oder vorzugsweise in Kombination mit geeigneten pharmazeutischen Trägerstoffen eingesetzt. Enthalten die pharmazeutischen Zubereitungen neben den erfindungsgemäßen Verbindungen pharmazeutische Trägerstoffe, beträgt der Wirkstoffgehalt dieser Mischungen 1 bis 95, vorzugsweise 10 bis 85, Gewichtsprozent der Gesamtmischung. Die Arzneimittel werden beispielsweise für die orale, rektale oder parenterale (intravenöse, intramusculäre, subcutane) Gabe in geeigneten Dosen formuliert. Die Tagesdosis liegt im allgemeinen für Säugetiere zwischen 0,01 und 35 mg/kg Körpergewicht, bevorzugt unter 10 mg/kg Körpergewicht. Eine zweckmäßige Tagesdosis für die Applikation an Menschen liegt zwischen 0,5 bis 5 mg/kg Körpergewicht.

Die pharmazeutischen Zubereitungen bestehen bevorzugt aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel als Süßungsmittel, als Geschmackskorrigenz, als Farbstoff oder als Konservierungsmittel dienen.

Neben den neuen Phenylaminothiophenessigsäuren können die pharmazeutischen Zubereitungen beispielsweise einen oder mehrere pharmakologisch aktive Bestandteile aus anderen Arzneimittelgruppen enthalten, beispielsweise

entzündungswidrig wirkende Corticosteroide (z. B. Prednison, Prednisolon, Dexamethason und deren Derivate);
Analgetika, wie beispielsweise Pyrazolonderivate, Propoxyphen,. Phenacetin, Salicylsäure-Derivate etc.;
Muskelrelaxantien, wie Pyridazin-Derivate, Carbamate (z. B. Phenprobamat) etc.;
Substanzen mit antiulcerogener Wirkung;
Antacida (wie beispielsweise Magnesiumtrisilikat und Aluminiumhydroxid);
lokal durchblutungsfördernde Substanzen, wie beispielsweise Nicotinsäurederivate und Dimethylsulfoxid;
Lokalanästhetica (wie beispielsweise Lidocain) und
Vitamine (wie beispielsweise Vitamin-$B_1$-chlorid-hydrochlorid, Vitamin-$B_6$-hydrochlorid, Vitamin-$B_{12}$-cyanokomplex und Thiamindisulfid).

Ein weiterer Gegenstand der Erfindung ist ein Verfahren zur Herstellung von Phenylaminothiophenessigsäuren der allgemeinen Formel I bzw. der Ausgestaltungen I* oder I**, worin $R^1$, $R^2$, $R^3$, $R^4$, $R^5$, $R^6$ und n bzw. die Substituenten der Ausgestaltungen die dort angegebene Bedeutung haben, und deren Salzen mit organischen und anorganischen Basen.

Das Verfahren ist dadurch gekennzeichnet, daß man funktionelle Carbonsäurederivate der allgemeinen Formel II

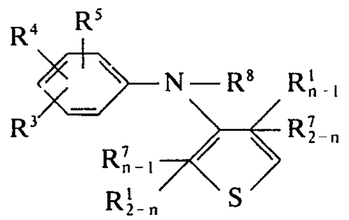

(II)

worin

$R^1$ ein Wasserstoffatom, ein Chloratom, ein Bromatom oder eine Methylgruppe und
$R^3$ ein Wasserstoffatom, ein Halogenatom, eine Alkylgruppe, eine Alkoxygruppe oder eine Trifluormethylgruppe bedeuten,
$R^4$ eine der Bedeutungen von $R^3$ hat,
$R^5$ ein Wasserstoffatom, ein Halogenatom oder eine Alkylgruppe,
$R^7$ eine $-CH_2-G$-Gruppe oder gemeinsam mit $R^8$ eine $-CH_2-CO$-Gruppe,
$R^8$ ein Wasserstoffatom oder gemeinsam mit $R^7$ eine $-CO-CH_2$-Gruppe,
G ein funktionelles Derivat einer Carbonsäuregruppe und
n 1 oder 2 bedeuten,

zu Verbindungen der allgemeinen Formel I oder deren Salzen lyolysiert, d. h. solvolytisch, hydrogenolytisch oder thermolytisch spaltet, und gegebenenfalls anschließend die erhaltenen Säuren bzw. Ester der allgemeinen Formel I in üblicher Weise ineinander und/oder in die Salze überführt.

Eine Ausgestaltung der Erfindung ist ein Verfahren zur Herstellung von Phenylaminothiophenessigsäuren der Formel I* und ihrer Salze, das dadurch gekennzeichnet ist, daß man funktionelle Phenylaminothiophenessigsäurederivate der allgemeinen Formel II*

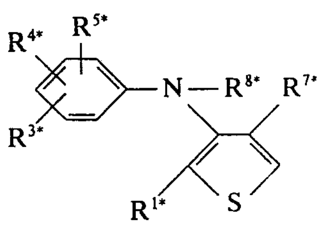

(II*)

worin

$R^{1*}$ ein Wasserstoffatom, ein Chloratom, ein Bromatom oder eine Methylgruppe und
$R^{3*}$ ein Wasserstoffatom, ein Chloratom, eine Methylgruppe oder eine Trifluormethylgruppe bedeuten,
$R^{4*}$ eine der Bedeutungen von $R^{3*}$ hat,

$R^{5*}$ ein Wasserstoffatom oder ein Chloratom,
$R^{7*}$ eine $-CH_2-G^*$-Gruppe oder gemeinsam mit $R^{8*}$ eine $-CH_2-CO$-Gruppe,
$R^{8*}$ ein Wasserstoffatom oder gemeinsam mit $R^{7*}$ eine $-CO-CH_2$-Gruppe und
$G^*$ ein funktionelles Derivat einer Carbonsäuregruppe bedeuten,

zu Verbindungen der allgemeinen Formel I* oder deren Salzen lyolysiert und gegebenenfalls anschließend die erhaltenen Säuren bzw. Ester der allgemeinen Formel I* halogeniert und/oder in üblicher Weise ineinander und/oder in die Salze überführt.

In einer bevorzugten Ausführungsform des Verfahrens geht man von Verbindungen der Formel II* aus, in denen G* eine Nitrilgruppe, eine Carbonsäurealkylestergruppe mit 1 bis 7 Kohlenstoffatomen im Alkylrest, eine Carbonsäurebenzylester- oder eine Carbonsäureamidgruppe darstellt.

Eine weitere Ausgestaltung der Erfindung ist ein Verfahren zur Herstellung von Phenylaminothiophenessigsäuren der Formel I** und ihrer Salze, das dadurch gekennzeichnet ist, daß man

a) funktionelle Phenylaminothiophenessigsäurederivate der allgemeinen Formel II**

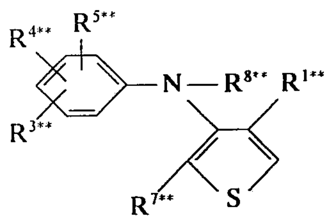

(II**)

worin

$R^{1**}$ ein Wasserstoffatom oder eine Methylgruppe und
$R^{3**}$ ein Wasserstoffatom, ein Chloratom, eine Methylgruppe oder eine Trifluormethylgruppe bedeuten,
$R^{4**}$ eine der Bedeutungen von $R^{3**}$ hat,
$R^{5**}$ ein Wasserstoffatom oder ein Chloratom,
$R^{7**}$ eine $-CH_2-G^{**}$-Gruppe oder gemeinsam mit $R^{8**}$ eine $-CH_2-CO$-Gruppe,
$R^{8**}$ ein Wasserstoffatom oder gemeinsam mit $R^{7**}$ eine $-CO-CH_2$-Gruppe und
$G^{**}$ ein funktionelles Derivat einer Carbonsäuregruppe bedeuten,

zu Verbindungen der allgemeinen Formel I** oder deren Salzen lyolysiert und gegebenenfalls anschließend die erhaltenen Säuren bzw. Ester der allgemeinen Formel I** in üblicher Weise ineinander und/oder in die Salze überführt oder

b) ein Phenylaminothiophenessigsäurederivat der allgemeinen Formel III**

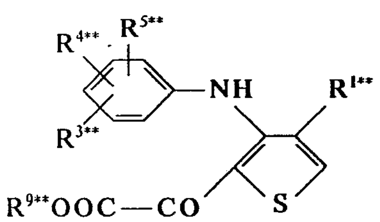

(III**)

worin

$R^{1**}$ ein Wasserstoffatom oder eine Methylgruppe und
$R^{3**}$ ein Wasserstoffatom, ein Chloratom, eine Methylgruppe oder eine Trifluormethylgruppe bedeuten,
$R^{4**}$ eine der Bedeutungen von $R^{3**}$ hat,
$R^{5**}$ ein Wasserstoffatom oder ein Chloratom,
$R^{9**}$ ein Wasserstoffatom oder eine Alkylgruppe mit 1 bis 5 Kohlenstoffatomen bedeuten,

oder ein Salz davon reduziert und gegebenenfalls anschließend die erhaltenen Säuren bzw. Ester der allgemeinen Formel I** in üblicher Weise ineinander und/oder in die Salze überführt.

In einer bevorzugten Ausführungsform des Verfahrens a) geht man von Verbindungen der Formel II** aus, in denen $R^{7**}$ eine $-CH_2-G^{**}$-Gruppe, $R^{8**}$ ein Wasserstoffatom und $G^{**}$ eine Nitrilgruppe, eine Carbonsäurealkylestergruppe mit 1 bis 7 Kohlenstoffatomen im Alkylrest, eine Carbonsäurebenzylester- oder eine Carbonsäureamidgruppe darstellen. Die Verfahrensvariante b)

ist gegenüber der Variante a) bevorzugt.

Unter der Lyolyse von Carbonsäurederivaten versteht man die solvolytische, hydrogenolytische oder thermolytische Spaltung. Als solvolytische Spaltungen kommen vor allem die Hydrolyse und die Alkoholyse in Frage. Zur hydrolytischen Spaltung von funktionellen Carbonsäurederivaten der allgemeinen Formel II, II* oder II** verwendet man ein wasserabgebendes Medium, das ganz oder teilweise aus Wasser bzw. aus bei den Reaktionsbedingungen wasser- oder OH-Ionen-abspaltenden Mitteln besteht. Die Reaktion kann als Homogenreaktion geführt werden, in welchem Fall man meist in Gegenwart eines polaren organischen Lösungsmittels oder eines Lösungsvermittlers arbeitet. Vorteilhafterweise werden als Lösungsmittel beispielsweise niedermolekulare Alkohole, Dioxan, Aceton, niedermolekulare Carbonsäuren, N-Methyl-pyrrolidon, Sulfolan oder Dimethylsulfoxid verwendet. Man kann aber die Reaktion auch als Heterogenreaktion durchführen. Der pH des wasserabgebenden Mediums richtet sich nach der chemischen Natur des eingesetzten Phenylaminothiophenessigsäurederivates, aber auch nach der Natur der gewünschten Verbindung der allgemeinen Formel I, I* bzw. I**; er kann daher neutral, sauer oder basisch sein. Er wird mit Säuren, Basen oder Puffern auf den gewünschten Wert eingestellt. Die Reaktionstemperaturen liegen zwischen 0° C und dem Siedepunkt des wasserabgebenden Mediums, im allgemeinen zwischen 0° und 150° C, insbesondere zwischen 20° und 120° C. Sie hängen im einzelnen auch davon ab, ob unter Druck oder bei Normaldruck gearbeitet wird. Die Reaktionszeiten liegen je nach Ansatz, Reaktionstemperaturen und übrigen Reaktionsparametern zwischen 10 Minuten und 20 Stunden. Nach vollzogener hydrolytischer Spaltung werden die Phenylaminothiophenessigsäuren nach üblichen Methoden, z. B. durch Umkristallisation oder durch Ansäuern ihrer Lösungen, gegebenenfalls unter Einengung ihrer Lösungen isoliert. Zu ihrer Reinigung kann deren alkalische Lösung mit einem organischen Lösungsmittel, das mit der alkalischen Lösung nicht mischbar ist, beispielsweise Ether, Benzol, Chlorbenzol, Chloroform oder Methylenchlorid, extrahiert werden.

Die Alkoholyse wird bevorzugt bei Carbonsäurederivaten der allgemeinen Formel II, II* oder II**, in denen G, G* bzw. G** eine Nitrilgruppe darstellt, durchgeführt. Man verwendet dazu ein Medium, das ganz oder teilweise aus dem betreffenden Alkohol und einem Protonendonator besteht, beispielsweise mit Salzsäuregas gesättigtes Methanol. Die Reaktionstemperaturen liegen zwischen −20° und 50° C, insbesondere zwischen 0° und 20° C. Die Reaktionszeiten liegen (je nach Reaktionstemperatur und übrigen Parametern) zwischen 30 Minuten und 10 Stunden. Nach vollzogener Alkoholyse werden die gebildeten Ester nach üblichen Methoden isoliert.

Die Lyolyse in Form der Hydrogenolyse wird bei Verbindungen der Formel II, II* bzw. II**, in denen G, G* bzw. G** eine Carbonsäurebenzylestergruppe darstellt, durchgeführt. Sie erfolgt unter den üblichen Bedingungen, beispielsweise mit Wasserstoff an Palladiumkohle oder Platin, bei −10° bis 50° C, vorzugsweise bei Raumtemperatur, unter 1−200, vorzugsweise 1−10 bar Druck und in einem inerten Lösungsmittel, wie Methanol, Essigsäureethylester oder vorzugsweise in Gegenwart von Eisessig.

Die Lyolyse in Form der Thermolyse wird bei Phenylaminothiophenessigsäurederivaten der Formel II, II* oder II** durchgeführt, in denen G, G* bzw. G** eine Carbonsäure-tert.-alkylestergruppe, vorzugsweise eine tert.-Butylestergruppe, darstellt.

Diese Reaktion wird unter den üblichen Bedingungen, gegebenenfalls in einem inerten organischen Lösungsmittel, beispielsweise Chlorbenzol, Xylol oder Toluol, ohne oder vorzugsweise in Gegenwart eines sauren Katalysators, beispielsweise p-Toluolsulfonsäure, durch Erhitzen auf 30 bis 200° C, vorzugsweise 70−150° C, durchgeführt.

Die Überführung der Phenylaminothiophenessigsäuren der allgemeinen Formel I, I* oder I** in ihre Salze kann durch direkte alkalische Solvolyse mit Hydroxylionen der Phenylaminothiophenessigsäurederivate der allgemeinen Formel II, II* oder II** erfolgen. Als alkalischer Reaktionspartner wird zweckmäßigerweise diejenige anorganische oder organische Base verwendet, deren Salz gewünscht wird. Man erhält die Salze aber auch, indem man die Phenylaminothiophenessigsäuren der allgemeinen Formel I, I* oder I** mit dem stöchiometrischen Äquivalent an entsprechender Base umsetzt oder leicht lösliche Salze durch doppelte Umsetzung in schwer lösliche Salze umwandelt oder beliebige Salze in pharmakologisch verträgliche Salze überführt.

Die als Ausgangs- oder Zwischenprodukte eingesetzten Phenylaminothiophenessigsäurederivate der Formel II, II* und II** sind ebenfalls neue Verbindungen. Sie werden nach bekannten Methoden hergestellt. So erhält man Nitrile der Formeln II, II* bzw. II** aus den entsprechenden Halogenmethylverbindungen, das heißt aus Verbindungen der Formel II, II* bzw. II**, in denen G ein Halogenatom, vorzugsweise ein Brom- oder Chloratom, bedeutet, durch Umsetzung mit Alkali- oder Erdalkalicyaniden, wie Natriumcyanid, Kaliumcyanid oder Bariumcyanid. Die Reaktion wird zweckmäßigerweise in einem aprotischen, dipolaren Lösungsmittel, z. B. Dimethylformamid, oder im Zweiphasensystem in Gegenwart eines Phasentransferkatalysators, wie Benzyltrimethylammoniumchlorid, bei Temperaturen von 0° bis 80° C durchgeführt.

Phenylaminothiophenessigsäureester der allgemeinen Formel II, II* und II** sind aus den Nitrilen durch Alkoholyse leicht zugänglich; ferner aus den freien Säuren der allgemeinen Formel I, I* und I** durch Umsetzung mit Alkoholen unter wasserabspaltenden Bedingungen oder durch Umsetzung von Säuren und Salzen mit Alkylierungsmitteln, z. B. Benzylester durch Umsetzung von Silbersalzen

mit Benzylhalogeniden.

Halogenmethylverbindungen erhält man durch Reduktion entsprechender Phenylaminothiophencarbonsäuren bzw. -carbonsäureester mit nachfolgender Halogenierung. Als Reduktionsmittel eignen sich beispielsweise Natriumboranat, Natrium/Ethanol oder Lithiumaluminiumhydrid. Die Reaktion wird vorzugsweise in inerten Lösungsmitteln, wie offenkettigen oder cyclischen Ethern, z. B. Diethylether oder Tetrahydrofuran, bei Temperaturen zwischen 0° und 25°C durchgeführt. Die entstandenen Hydroxymethylverbindungen werden durch Umsetzung mit Halogenwasserstoffen, wie Chlor- oder Bromwasserstoff, in die Halogenmethylverbindungen übergeführt. Die Reaktion erfolgt in polarem, vorzugsweise wäßrigem Medium, z. B. in konzentrierter Salzsäure, bei Temperaturen zwischen 10 und 50°C.

4-Phenylaminothiophen-3-carbonsäuren erhält man gemäß DE-PS 1 493 705. Die entsprechenden Ester werden durch Umsetzung dieser Säuren mit Alkoholen in an sich bekannter Weise erhalten. 3-Phenylaminothiophen-2-carbonsäuren oder ihre Ester werden in Analogie zu DE-PS 1 493 705 durch Umsetzung von 3-Oxo-tetrahydrothiophen-2-carbonsäuremethylester oder -ethylester [E. E. Moore and M. B. Moore, J. Am. Chem. Soc. 68 (1946) 910; Erastov, O. A., Ignat'eva, S. N., Khim. Geterotsikl. Soedin. 1971, 7 (11) 1473–80] mit durch $R^3$, $R^4$, $R^5$ substituierten Anilinen, anschließende Dehydrierung und gegebenenfalls Hydrolyse erhalten.

Die gegebenenfalls sich anschließende Halogenierung von erhaltenen Verbindungen der allgemeinen Formel I* erfolgt nach an sich bekannten Verfahren. Als Halogenierungsmittel für die Halogenierung des Thiophenrings kommen beispielsweise die freien Halogene Chlor oder Brom, N-Halogenamide und -imide, Sulfurylchlorid oder Phosphorpentachlorid in Frage. Die Umsetzung mit dem Halogenierungsmittel kann in bekannter Weise, z. B. in einem inerten, nicht wäßrigen Lösungsmittel, wie einem chlorierten Kohlenwasserstoff oder Eisessig (z. B. mit Chlor, Brom oder Sulfurylchlorid), bei Temperaturen von −40°C bis 20°C, vorzugsweise bei −20° bis 0°C, mit überschüssigen oder vorzugsweise äquivalenten Mengen des Halogenierungsmittels durchgeführt werden. Die Umsetzung ist in der Regel 10 Minuten bis 2 Stunden nach Zugabe des Halogenierungsmittels beendet.

Die Hydrolyse von Phenylaminothiophenessigsäurelactamen der allgemeinen Formel II, II* oder II**, d. h. von Verbindungen, in denen $R^7$, $R^{7*}$ bzw. $R^{7**}$ gemeinsam mit $R^8$, $R^{8*}$ bzw. $R^{8**}$ eine $-CH_2-CO-$Gruppe und $R^8$, $R^{8*}$ bzw. $R^{8**}$ gemeinsam mit $R^7$, $R^{7*}$ bzw. $R^{7**}$ eine $-CO-CH_2-$Gruppe bedeuten, erfolgt in an sich bekannter Weise durch alkalische oder saure Verseifung in polaren Lösungsmitteln, wie Alkoholen, z. B. Methanol, bei Temperaturen von 20°C bis zur Siedetemperatur des Lösungsmittels.

Die Reaktion ist — je nach Reaktionstemperatur — im allgemeinen nach 2 Stunden beendet.

Die Reduktion der Phenylaminothiophenessigsäurederivate der allgemeinen Formel III** erfolgt in an sich bekannter Weise durch Umsetzung mit einem geeigneten Reduktionsmittel, z. B. Natriumborhydrid, in inerten Lösungsmitteln, wie Tetrahydrofuran oder Dimethylformamid, bei Temperaturen von 0° bis zur Siedetemperatur des Lösungsmittels, vorzugsweise bei 50° bis 80°C. Die Reaktionsdauer beträgt, je nach Temperatur, 1 bis 5 Stunden.

Verbindungen der Formel III** erhält man durch Umsetzung von entsprechenden 3-Phenylaminothiophenen mit Oxalsäurealkylesterchloriden und gegebenenfalls anschließende Verseifung.

Die Umsetzung erfolgt in Gegenwart einer Hilfsbase, wie Pyridin oder Triethylamin, in inerten Lösungsmitteln, wie cyclischen oder offenkettigen Ethern, z. B. Tetrahydrofuran oder Diethylether, bei Temperaturen zwischen 0° und 50°C, vorzugsweise bei Raumtemperatur.

Zu 3-Phenylaminothiophenen gelangt man durch Decarboxylierung von 4-Phenylamino-3-thiophencarbonsäuren, beispielsweise durch Erhitzen auf Temperaturen von 120° bis 200°C.

Die Hydroxymethyl- und Halogenmethylverbindungen als Rohprodukte sind relativ instabile Verbindungen; sie werden daher zweckmäßigerweise nach Erhalt umgehend weiter umgesetzt, wobei gegebenenfalls auf Reinigungsoperationen verzichtet wird.

Die folgenden Beispiele erläutern die Erfindung näher. Die Abkürzung F. bzw. Kp. bedeuten Schmelzpunkt bzw. Siedepunkt. Z. Bedeutet Zersetzungspunkt.

## Beispiel 1

31 g (0,11 Mol) 4-(2,6-Dichloranilino)-3-thiophenacetonitril werden in einer Lösung von 110 g Natriumhydroxid in 450 ml Wasser und 200 ml Methanol 4 Stunden gekocht. Man verdünnt mit 2 Liter warmem Wasser, extrahiert mit Toluol, säuert die wässerige Phase an und saugt den entstandenen Niederschlag von 4-(2,6-Dichloranilino)-3-thiophenessigsäure ab. Der Niederschlag wird mit Diisopropylether ausgekocht, getrocknet und schmilzt danach bei 179–180°C.

Analog erhält man aus:

4-(2-Chloranilino)-3-thiophenacetonitril,
4-(2,6-Dimethylanilino)-3-thiophenacetonitril,
4-(2-Chlor-6-methylanilino)-3-thiophenacetonitril,
4-(4-Methoxy-2-methylanilino)-3-thiophenacetonitril,

4-(2-Chlor-3-methylanilino)-3-thiophenacetonitril bzw.
4-(3-Trifluormethylanilino)-3-thiophenacetonitril,

durch Kochen mit methanolischer Natriumhydroxid-Lösung und entsprechende Aufarbeitung

4-(2-Chloranilino)-3-thiophenessigsäure (F. 125—126,5°C),
4-(2,6-Dimethylanilino)-3-thiophenessigsäure (F. 152,5—153°C),
4-(2-Chlor-6-methylanilino)-3-thiophenessigsäure (F. 168—169°C),
4-(4-Methoxy-2-methylanilino)-3-thiophenessigsäure,
4-(2-Chlor-3-methylanilino)-3-thiophenessigsäure (F. 105—106°C) bzw.
4-(3-Trifluormethylanilino)-3-thiophenessigsäure (F. 108—109°C).

Beispiel 2

26,3 g (0,1 Mol) 4-(3-Chlor-2-methylanilino)-3-thiophenacetonitril werden in 135 ml konzentrierte Schwefelsäure eingetragen, 1 Stunde bei 20°C gerührt, mit 49 ml Wasser tropfenweise versetzt und 3 Stunden auf 100°C erhitzt. Man gießt auf Eis und saugt den entstandenen Niederschlag ab. Umkristallisation aus Diisopropylether liefert 4-(3-Chlor-2-methylanilino)-3-thiophenessigsäure. F. 126 bis 128°C.

Analog erhält man aus:

4-(2,6-Dichlor-3-methylanilino)-3-thiophenacetonitril,
4-(2-Chlor-3-methylanilino)-3-thiophenacetonitril,
4-(2,6-Difluoranilino)-3-thiophenacetonitril,
4-(2,6-Dimethylanilino)-3-thiophenacetonitril,
5-Chlor-4-(2,6-dichloranilino)-3-thiophenacetonitril, (Beispiel 12)
4-(2-Chloranilino)-5-methyl-3-thiophenacetonitril bzw.
4-(2,6-Dichloranilino)-5-methyl-3-thiophenacetonitril

durch Solvolyse mit konzentrierter Schwefelsäure

4-(2,6-Dichlor-3-methylanilino)-3-thiophenessigsäure (F. 159—160°C),
4-(2-Chlor-3-methylanilino)-3-thiophenessigsäure (F. 105—106°C),
4-(2,6-Difluoranilino)-3-thiophenessigsäure (F. 151°C),
4-(2,6-Dimethylanilino)-3-thiophenessigsäure (F. 152,5—153°C),
5-Chlor-4-(2,6-dichloranilino)-3-thiophenessigsäure (Z. 141—142°C),
4-(2-Chloranilino)-5-methyl-3-thiophenessigsäure (F. 196°C) bzw.
4-(2,6-Dichloranilino)-5-methyl-3-thiophenessigsäure (F. 155—156°C).

Beispiel 3

31 g (0,11 Mol) 4-(2,6-Dichloranilino)-3-thiophenacetonitril werden in 30 ml Methanol gelöst. Die Lösung wird bei 10°C mit Chlorwasserstoffgas gesättigt und vier Stunden bei 10°C stehengelassen. Anschließend wird auf 200 ml Wasser gegossen und mit 100 ml Diethylther extrahiert, die etherische Phase wird mit 50 ml Wasser gewaschen, getrocknet und eingeengt. Man erhält als Öl 4-(2,6-Dichloranilino)-3-thiophenessigsäuremethylester.

Analog erhält man durch Solvolyse mit Ethanol (anstelle von Methanol) 4-(2,6-Dichloranilino)-3-thiophenessigsäureethylester (F. 64—65°C).

Analog erhält man aus 4-(2-Chlor-6-methylanilino)-3-thiophenacetonitril durch Solvolyse mit methanolischer Salzsäure 4-(2-Chlor-6-methylanilino)-3-thiophenessigsäuremethylester (F. 90—100°C).

Beispiel 4

Die in den Beispielen 1—3 aufgeführten Nitrile erhält man auf folgendem Weg:

a) 29,2 g (0,1 Mol) 3-(2,6-Dichloranilino)-4-chlormethylthiophen und 13 g (0,2 Mol) Kaliumcyanid werden in 250 ml Dimethylformamid und 0,5 ml Wasser gelöst und eine Stunde auf 45°C erwärmt. Man gießt auf Eis, extrahiert mit Essigsäureethylester, wäscht die Essigesterphase mit Wasser, trocknet über Natriumsulfat und engt ein. Als öliger Rückstand bleibt 4-(2,6-Dichloranilino)-3-thiophenacetonitril, der beim Stehen auskristallisiert (F. 81—83°C).

Analog erhält man durch Umsetzung von

3-(2-Chloranilino)-4-chlormethylthiophen,
3-Chlormethyl-4-(2-chlor-6-methylanilino)-thiophen,
3-Chlormethyl-4-(2-chlor-3-methylanilino)-thiophen,
3-Chlormethyl-4-(2,6-dichlor-3-methylanilino)-thiophen,
3-Chlormethyl-4-(3-chlor-2-methylanilino)-thiophen,
3-Chlormethyl-4-(2,6-difluoranilino)-thiophen,
3-(2-Chloranilino)-4-chlormethyl-2-methylthiophen bzw.
4-Chlormethyl-3-(2,6-dichloranilino)-2-methylthiophen

mit Kaliumcyanid und entsprechende Aufarbeitung

4-(2-Chloranilino)-3-thiophenacetonitril (Öl),
4-(2-Chlor-6-methylanilino)-3-thiophenacetonitril (Öl),
4-(2-Chlor-3-methylanilino)-3-thiophenacetonitril,
4-(2,6-Dichlor-3-methylanilino)-3-thiophenacetonitril (Öl),
4-(3-Chlor-2-methylanilino)-3-thiophenacetonitril (Öl),
4-(2,6-Difluoranilino)-3-thiophenacetonitril (Öl),
4-(2-Chloranilino)-5-methyl-3-thiophenacetonitril (Öl) bzw.
4-(2,6-Dichloranilino)-5-methyl-3-thiophenacetonitril (F. 115° C).

Alternativ erhält man die Ausgangsnitrile in folgender Weise:
2,72 g (10 mMol) 3-Chlormethyl-4-(2-chlor-3-methylanilino)-thiophen in 15 ml Dimethylsulfoxid werden zu einer Lösung von 650 mg Natriumcyanid in 15 ml Dimethylsulfoxid bei 40° C zugetropft. Man rührt noch drei Stunden, gießt auf Wasser und extrahiert dreimal mit Ethylacetat. Die vereinigten Extrakte werden mit je 50 ml 6 n-Salzsäure und Wasser gewaschen, getrocknet und eingeengt. Man erhält als Öl rohes 4-(2-Chlor-3-methylanilino)-3-thiophenacetonitril.
Analog erhält man durch Umsetzung von

3-Chlormethyl-4-(2,6-dimethylanilino)-thiophen,
3-Chlormethyl-4-(4-methoxy-2-methylanilino)-thiophen bzw.
3-Chlormethyl-4-(3-trifluormethylanilino)-thiophen

mit Natriumcyanid

4-(2,6-Dimethylanilino)-3-thiophenacetonitril,
4-(4-Methoxy-2-methylanilino)-3-thiophenacetonitril bzw.
4-(3-Trifluormethylanilino)-3-thiophenacetonitril.

b) Zu den Chlormethylverbindungen gelangt man auf folgendem Weg:
27,4 g (0,1 Mol) 4-(2,6-Dichloranilino)-3-thiophenmethanol werden in 800 ml konzentrierter wässeriger Salzsäure gelöst, acht Stunden stehengelassen, auf Eis gegossen und mit Toluol extrahiert. Die organische Phase wird mit Natriumhydrogencarbonatläsung gewaschen, getrocknet und eingeengt. Umkristallisation des Rückstandes aus Petrolether liefert 3-(2,6-Dichloranilino)-4-chlormethylthiophen (F. 89—92° C).
Analog erhält man durch Umsetzung von

4-(2-Chloranilino)-3-thiophenmethanol,
4-(2,6-Dimethylanilino)-3-thiophenmethanol,
4-(2-Chlor-6-methylanilino)-3-thiophenmethanol,
4-(4-Methoxy-2-methylanilino)-3-thiophenmethanol,
4-(2-Chlor-3-methylanilino)-3-thiophenmethanol,
4-(3-Trifluormethylanilino)-3-thiophenmethanol,
4-(2,6-Dichlor-3-methylanilino)-3-thiophenmethanol,
4-(3-Chlor-2-methylanilino)-3-thiophenmethanol,
4-(2,6-Difluoranilino)-3-thiophenmethanol,
4-(2-Chloranilino)-5-methyl-3-thiophenmethanol bzw.
4-(2,6-Dichloranilino)-5-methyl-3-thiophenmethanol

mit wässeriger Salzsäure

3-(2-Chloranilino)-4-chlormethyl-thiophen,
3-Chlormethyl-4-(2,6-dimethylanilino)-thiophen (F. 149° C),
3-Chlormethyl-4-(2-chlor-6-methylanilino)-thiophen,

3-Chlormethyl-4-(4-methoxy-2-methylanilino)-thiophen,
3-Chlormethyl-4-(2-chlor-3-methylanilino)-thiophen (F. 60—63°C),
3-Chlormethyl-4-(3-trifluormethylanilino)-thiophen,
3-Chlormethyl-4-(2,6-dichlor-3-methylanilino)-thiophen (F. 68—70°C),
3-Chlormethyl-4-(3-chlor-2-methylanilino)-thiophen,
3-Chlormethyl-4-(2,6-difluoranilino)-thiophen,
3-(2-Chloranilino)-4-chlormethyl-2-methylthiophen (Öl) bzw.
4-Chlormethyl-3-(2,6-dichloranilino)-2-methylthiophen (F. 63—65°C).

c) Die Methanole werden auf folgende Weise hergestellt:
57,63 g (0,2 Mol) 4-(2,6-Dichloranilino)-3-thiophencarbonsäure (in 350 ml Tetrahydrofuran gelöst) werden zu einer Suspension von 7,59 g (0,2 Mol) Lithiumaluminiumhydrid in 170 ml abs. Tetrahydrofuran unter Rühren und Stickstoff bei 15°C innerhalb einer Stunde zugetropft. Anschließend wird eine Stunde bei Raumtemperatur weitergerührt. Das Reaktionsgemisch wird mit 2 Liter eiskaltem Wasser hydrolysiert, mit verdünnter Schwefelsäure auf pH 5 angesäuert und mit 1,5 Liter Ethylacetat extrahiert. Der Extrakt wird mit 100 ml 2 n-Natriumhydrogencarbonatlösung gewaschen, über Natriumsulfat getrocknet und eingeengt, wobei in nahezu quantitativer Ausbeute 4-(2,6-Dichloranilino)-3-thiophenmethanol als viskoses Öl anfällt, das langsam erstarrt. Umkristallisation aus Cyclohexan liefert reines 4-(2,6-Dichloranilino)-3-thiophenmethanol (F. 96—97°C).
Analog erhält man durch Reduktion von

4-(2-Chloranilino)-3-thiophencarbonsäure,
4-(2,6-Dimethylanilino)-3-thiophencarbonsäure,
4-(2-Chlor-6-methylanilino)-3-thiophencarbonsäure,
4-(4-Methoxy-2-methylanilino)-3-thiophencarbonsäure,
4-(2-Chlor-3-methylanilino)-3-thiophencarbonsäure bzw.
4-(3-Trifluormethylanilino)-3-thiophencarbonsäure

mit Lithiumaluminiumhydrid

4-(2-Chloranilino)-3-thiophenmethanol (Öl),
4-(2,6-Dimethylanilino)-3-thiophenmethanol (F. 87°C),
4-(2-Chlor-6-methylanilino)-3-thiophenmethanol (F. 93—95°C),
4-(4-Methoxy-2-methylanilino)-3-thiophenmethanol,
4-(2-Chlor-3-methylanilino)-3-thiophenmethanol bzw.
4-(3-Trifluormethylanilino)-3-thiophenmethanol.

Analog erhält man durch Reduktion von

4-(2,6-Dichlor-3-methylanilino)-3-thiophencarbonsäuremethylester,
4-(3-Chlor-2-methylanilino)-3-thiophencarbonsäuremethylester,
4-(2,6-Difluoranilino)-3-thiophencarbonsäuremethylester,
4-(2-Chloranilino)-5-methyl-3-thiophencarbonsäuremethylester bzw.
4-(2,6-Dichloranilino)-5-methyl-3-thiophencarbonsäuremethylester

mit Lithiumaluminiumhydrid

4-(2,6-Dichlor-3-methylanilino)-3-thiophenmethanol (F. 121—122°C),
4-(3-Chlor-2-methylanilino)-3-thiophenmethanol (Öl),
4-(2,6-Difluoranilino)-3-thiophenmethanol (F. 124—126°C),
4-(2-Chloranilino)-5-methyl-3-thiophenmethanol (Öl) bzw.
4-(2,6-Dichloranilino)-5-methyl-3-thiophenmethanol (F. 64—65°C).

d) Zu den Anilinothiophencarbonsäuren bzw. -thiophencarbonsäureestern gelangt man auf folgendem Weg:
15,9 g (0,1 Mol) 4-Oxo-tetrahydro-3-thiophencarbonsäuremethylester, 17,5 g 2,6-Dichloranilin und 200 mg p-Toluolsulfonsäure werden in 500 ml Dichlorethan sechs Stunden am Wasserabscheider erhitzt. Man kühlt die Lösung auf −25°C ab und versetzt tropfenweise mit 52,5 g Brom in 100 ml Dichlorethan. Man rührt weitere 5 Minuten, versetzt mit 100 ml 10%iger Natriumsulfitlösung, trennt die organische Phase ab, wäscht mit Wasser und Sodalösung, trocknet und engt ein. Man erhält den reinen 4-(2,6-Dichloranilino)-3-thiophencarbonsäuremethylester nach Kugelrohrdestillation (Kp. 140°/$10^{-2}$ Pascal).

Analog erhält man aus

4-Oxo-tetrahydro-3-thiophencarbonsäuremethylester und
2,6-Dichlor-3-methylanilin,
3-Chlor-2-methylanilin bzw.
2,6-Difluoranilin

als Produkte

4-(2,6-Dichlor-3-methylanilino)-3-thiophencarbonsäuremethylester,
4-(3-Chlor-2-methylanilino)-3-thiophencarbonsäuremethylester (F. 80—82° C) bzw.
4-(2,6-Difluoranilino)-3-thiophencarbonsäuremethylester (F. 90—92° C).

Ebenso erhält man aus

5-Methyl-4-oxo-tetrahydro-3-thiophencarbonsäuremethylester und
2-Chloranilin bzw.
2,6-Dichloranilin
4-(2-Chloranilino)-5-methyl-3-thiophencarbonsäuremethylester (F. 64—65° C) bzw.
4-(2,6-Dichloranilino)-5-methyl-3-thiophencarbonsäuremethylester (F. 86° C) bzw. aus
4-Methyl-3-oxo-tetrahydro-2-thiophencarbonsäuremethylester und
2,6-Dichloranilin
3-(2,6-Dichloranilino)-4-methyl-2-thiophencarbonsäuremethylester (F. 110—112° C).

Zur Herstellung der Anilinothiophencarbonsäure kann auch folgendermaßen vorgegangen werden:
15,9 g (0,1 Mol) 3-Oxo-tetrahydro-2-thiophencarbonsäuremethylester, 14,2 g 2-Chlor-3-methylanilin und 200 mg p-Toluolsulfonsäure werden in 500 ml Toluol sechs Stunden am Wasserabscheider erhitzt. Anschließend fügt man 24,6 g (0,1 Mol) Chloranil zu und kocht noch eine Stunde lang. Man kühlt ab, filtriert, wäscht das Filtrat mit je 100 ml 2 n-Natronlauge, Natriumdithionitlösung und Wasser, engt ein und reinigt den Rückstand säulenchromatographisch (Kieselgel, Toluol/Essigester 4/1). Man erhält 3-(2-Chlor-3-methylanilino)-2-thiophencarbonsäuremethylester als Öl.
Analog erhält man aus

3-Oxo-tetrahydro-2-thiophencarbonsäuremethylester und
2,6-Dichloranilin,
2,4-Dichlor-5-methylanilin bzw.
2-Chlor-5-methylanilin

die folgenden Verbindungen

3-(2,6-Dichloranilino)-2-thiophencarbonsäuremethylester,
3-(2,4-Dichlor-5-methylanilino)-2-thiophencarbonsäuremethylester bzw.
3-(2-Chlor-5-methylanilino)-2-thiophencarbonsäuremethylester.

Die Verseifung der Anilinothiophencarbonsäureester wird wie folgt durchgeführt:
3,16 g (0,1 Mol) 3-(2,6-Dichloranilino)-4-methyl-2-thiophencarbonsäuremethylester werden in 300 ml Methanol und 100 ml 6 n-Kalilauge sechs Stunden gekocht. Anschließend wird Methanol abdestilliert, mit Wasser aufgenommen und mit Toluol gewaschen. Die wässerige Phase wird angesäuert und mit Ether extrahiert. Die organische Phase wird getrocknet, mit Bleicherde (z. B. Tonsil[R]) geklärt und eingeengt. Umkristallisation aus Toluol liefert 3-(2,6-Dichloranilino)-4-methyl-2-thiophencarbonsäure (F. 157—158° C).
Analog erhält man aus

4-(2-Chloranilino)-3-thiophencarbonsäuremethylester,
4-(2,6-Dimethylanilino)-3-thiophencarbonsäuremethylester,
4-(2-Chlor-6-methylanilino)-3-thiophencarbonsäuremethylester,
4-(4-Methoxy-2-methylanilino)-3-thiophencarbonsäuremethylester,
4-(2-Chlor-3-methylanilino)-3-thiophencarbonsäuremethylester bzw.
4-(3-Trifluormethylanilino)-3-thiophencarbonsäuremethylester

durch Verseifung

4-(2-Chloranilino)-3-thiophencarbonsäure,
4-(2,6-Dimethylanilino) 3-thiophencarbonsäure,
4-(2-Chlor-6-methylanilino)-3-thiophencarbonsäure,

4-(4-Methoxy-2-methylanilino)-3-thiophencarbonsäure,
4-(2-Chlor-3-methylanilino)-3-thiophencarbonsäure bzw.
4-(3-Trifluormethylanilino)-3-thiophencarbonsäure.

e) Die Oxo-tetrahydro-thiophencarbonsäuren sind bekannt oder werden wie folgt hergestellt: 100 g (1 Mol) Methacrylsäuremethylester werden zu 106 g Thioglykolsäuremethylester und 1 ml Piperidin zugetropft. Das Reaktionsgemisch wird zu einer Lösung von 40 g Natriummethylat in 200 ml Methanol zugetropft. Man rührt eine weitere halbe Stunde, gießt auf Wasser, säuert an, extrahiert mit Methylenchlorid, wäscht die organische Phase mit Wasser und trocknet mit Natriumsulfat. Nach der Destillation erhält man 4-Methyl-3-oxo-tetrahydro-2-thiophencarbonsäuremethylester (Kp 110 bis 115°C/14 Pascal).

Analog erhält man aus Acrylsäuremethylester und Thiomilchsäuremethylester 5-Methyl-4-oxo-tetrahydro-3-thiophencarbonsäuremethylester (Kp. 112°C/14 Pascal).

Beispiel 5

0,32 g (1 mMol) 3-(2-Chlor-6-methylanilino)-2-thiophenglyoxylsäureethylester und 1 g Natriumboranat werden in 10 ml Dimethylformamid 2 Stunden auf 80°C erwärmt. Anschließend gießt man auf Wasser, wäscht mit Ether, säuert die wässerige Phase an und extrahiert mit Essigester. Einengen der organischen Phase und Säulenchromatographie (Kieselgel/Methylenchlorid) liefert in der ersten Fraktion 3-(2-Chlor-6-methylanilino)-2-thiophenessigsäureethylester.

Analog erhält man aus 3-(2,6-Dichloranilino)-2-thiophenglyoxysäureethylester durch Reduktion mit Natriumboranat 3-(2,6-Dichloranilino)-2-thiophenessigsäureethylester.

Beispiel 6

3,16 g (10 mMol) 3-(2,6-Dichloranilino)-2-thiophenglyoxylsäure und 2 g Natriumboranat werden in 10 ml Dimethylformamid 4 Stunden auf 80°C erwärmt. Man gießt auf Wasser, extrahiert mit Toluol, säuert die wässerige Phase an und extrahiert mit Essigester, trocknet die organische Phase und engt ein. Umkristallisation aus Toluol liefert 3-(2,6-Dichloranilino)-2-thiophenessigsäure (F. 164—168°C).

Analog erhält man aus

3-(2-Chlor-6-methylanilino)-2-thiophenglyoxylsäure bzw.
3-(2,6-Dichloranilino)-4-methyl-2-thiophenglyoxylsäure

durch Reduktion mit Natriumboranat und entsprechende Aufarbeitung

3-(2-Chlor-6-methylanilino)-2-thiophenessigsäure (F. 147—149°C) bzw.
3-(2,6-Dichloranilino)-4-methyl-2-thiophenessigsäure (F. 126—128°C).

Die Ausgangsverbindungen erhält man auf folgende Weise:

a) 3,44 g (10 mMol) 3-(2,6-Dichloranilino)-2-thiophenglyoxylsäureethylester werden in 10 ml Ethanol mit 1 g Kaliumhydroxid gerrührt, bis nach 10 Minuten eine homogene Lösung entstanden ist. Man verdünnt mit 50 ml Wasser, säuert an, extrahiert mit Essigester, trocknet die organische Phase und engt ein. Umkristallisation aus Cyclohexan mit wenig Toluol liefert 3-(2,6-Dichloranilino)-2-thiophenglyoxylsäure.

Analog erhält man aus

3-(2-Chlor-6-methylanilino)-2-thiophenglyoxylsäureethylester bzw.
3-(2,6-Dichloranilino)-4-methyl-2-thiophenglyoxylsäureethylester

durch Verseifen mit ethanolischer Kalilauge

3-(2-Chlor-6-methylanilino)-2-thiophenglyoxylsäure (F. 127—136°C) bzw.
3-(2,6-Dichloranilino)-4-methyl-2-thiophenglyoxylsäure (F. 126—128°C).

b) 2,44 g (10 mMol) 3-(2,6-Dichloranilino)-thiopen, 1,01 g Triethylamin und 1,37 g (10 mMol) Chloroxalsäureethylester werden in 20 ml Tetrahydrofuran 10 Stunden bei Raumtemperatur gerührt. Man versetzt mit je 100 ml Wasser und Essigester, trocknet die organische Phase und engt ein. Umkristallisation aus Ethanol liefert 3-(2,6-Dichloranilino)-2-thiophenglyoxylsäureethylester.

Analog erhält man aus

3-(2-Chlor-6-methylanilino)-thiophen bzw.
3-(2,6-Dichloranilino)-4-methylthiophen und
Chloroxalsäureethylester
3-(2-Chlor-6-methylanilino)-2-thiophenglyoxylsäureethylester (F. 155—156° C) bzw.
3-(2,6-Dichloranilino)-4-methyl-2-thiophenglyoxylsäureethylester.

c) 2,88 g (10 mMol) 4-(2,6-Dichloranilino)-3-thiophencarbonsäure (siehe Beispiel 4d) werden bei 16 Pascal und 150° C in einer Kugelrohrdestillationsapparatur decarboxyliert. Als Destillat erhält man reines 3-(2,6-Dichloranilino)-thiophen.

Analog erhält man aus

4-(2-Chlor-6-methylanilino)-3-thiophencarbonsäure bzw.
3-(2,6-Dichloranilino)-4-methyl-2-thiophencarbonsäure
3-(2-Chlor-6-methylanilino)-thiophen bzw.
3-(2,6-Dichloranilino)-4-methylthiophen.

Beispiel 7

316,2 g (1 mMol) 4-(2,6-Dichloranilino)-3-thiophenessigsäuremethylester werden in einer Lösung von 11 ml 2 n-Natronlauge in 4,5 ml Wasser und 3 ml Methanol eine Stunde gekocht. Anschließend wird mit 20 ml Wasser verdünnt, mit 20 ml Methylenchlorid extrahiert und die wässerige Phase angesäuert. Der entstandene Niederschlag wird abgesaugt und mit 5 ml Diisopropylether gewaschen.

Man erhält 4-(2,6-Dichloranilino)-3-thiophenessigsäure (F. 179—180° C).

Analog erhält man aus 5-Chlor-4-(2-chlor-6-methylanilino)-3-thiophenessigsäuremethylester durch Kochen mit methanolischer Natronlauge und entsprechende Aufarbeitung 5-Chlor-4-(2-chlor-6-methylanilino)-3-thiophenessigsäure (F. 143—146° C).

Beispiel 8

330 mg (1 mMol) 3-(2,6-Dichloranilino)-2-thiophen-essigsäureethylester werden mit 1 g Kaliumhydroxid in 5 ml Ethanol eine Stunde gekocht. Anschließend wird mit 20 ml Wasser verdünnt und mit 20 ml Methylenchlorid extrahiert. Die wässerige Lösung wird angesäuert, der entstandene Niederschlag wird abgesaugt und mit 3 ml Diisopropylether gewaschen. Man erhält 3-(2,6-Dichloranilino)-2-thiophenessigsäure (F. 164—168° C).

Analog erhält man aus

3-(2-Chlor-6-methylanilino)-2-thiophenessigsäureethylester,
3-(2,6-Dichloranilino)-2-thiophenessigsäuremethylester bzw.
5-Chlor-4-(2-chlor-6-methylanilino)-3-thiophenessigsäuremethylester

durch Kochen mit ethanolischer Kalilauge und entsprechende Aufarbeitung

3-(2-Chlor-6-methylanilino)-2-thiophenessigsäure (F. 147—149° C),
3-(2,6-Dichloranilino)-2-thiophenessigsäure (F. 164—168° C) bzw.
5-Chlor-4-(2-chlor-6-methylanilino)-3-thiophenessigsäure (F. 143—146° C).

Beispiel 9

357 mg (1 mMol) 4-(2,6-Dichloranilino)-3-thiophenessigsäure-tert.-butylester werden mit 10 mg p-Toluolsulfonsäure in Toluol 2 Stunden zum Sieden erhitzt. Man wäscht die Reaktionslösung mit Wasser, trocknet, engt ein und erhält 4-(2,6-Dichloranilino)-3-thiophenessigsäure (F. 179—180° C).

Die Ausgangsverbindung erhält man auf folgende Weise:

316 mg (1 mMol) 4-(2,6-Dichloranilino)-3-thiophenessigsäuremethylester werden in 20 ml tert.-Butanol und 2 ml konzentrierter Schwefelsäure 2 Stunden auf 60° C erwärmt. Der überschüssige Alkohol wird abdestilliert. Chromatographie über eine Säule (Kieselgel/Methylenchlorid) liefert in der ersten Fraktion 4-(2,6-Dichloranilino)-3-thiophenessigsäure-tert.-butylester.

Beispiel 10

393 mg (1 mMol) 4-(2,6-Dichloranilino)-3-thiophenessigsäurebenzylester werden in Gegenwart von 50 mg 10%iger Palladium/Aktivkohle bei Raumtemperatur und Atmosphärendruck in einem Gemisch aus 100 ml Essigester und 10 ml Eisessig in einer Umlaufhydrierapparatur bis zur Aufnahme der theoretischen Menge Wasserstoff hydrogenolysiert. Man filtriert vom Katalysator ab, engt das Filtrat im Vakuum ein und löst den Rückstand in 1 n-Natronlauge auf. Man extrahiert die wässerige Lösung mit Toluol, säuert mit 1 n-Salzsäure an und extrahiert wieder mit Toluol. Die organische Phase wird getrocknet und eingeengt. Man erhält 4-(2,6-Dichloranilino)-3-thiophenessigsäure (F. 179–180° C).

Die Ausgangsverbindung erhält man auf folgende Weise:

316 mg (1 mMol) 4-(2,6-Dichloranilino)-3-thiophenessigsäuremethylester werden in eine Lösung von 10 mg Kaliumbenzylalkoholat in 20 ml Benzylalkohol eingetragen. Man rührt 2 Stunden bei Raumtemperatur, destilliert im Vakuum überschüssigen Alkohol ab und erhält als Rückstand (4-(2,6-Dichloranilino)-3-thiophenessigsäurebenzylester.

Beispiel 11

30,1 g (0,1 Mol) 4-(2,6-Dichloranilino)-3-thiophenacetamid werden in 135 ml konzentrierter Schwefelsäure und 49 ml Wasser 3 Stunden auf 100° C erwärmt. Man gießt auf Eis und saugt den entstandenen Niederschlag von 4-(2,6-Dichloranilino)-3-thiophenessigsäure ab. Der Niederschlag wird mit Diisopropylether ausgekocht, getrocknet und schmilzt danach bei 179–180° C.

Die Ausgangsverbindung erhält man auf folgende Weise:

28,3 g (0,1 Mol) 4-(2,6-Dichloranilino)-3-thiophenacetonitril werden in 150 ml konzentrierter Schwefelsäure eingetragen und eine Stunde bei Raumtemperatur gerührt. Man gießt auf Eis und saugt den entstandenen Niederschlag ab. Umkristallisation aus Essigester liefert 4-(2,6-Dichloranilino)-3-thiophenacetamid (F. 172–173° C).

Beispiel 12

Zu einer Lösung von 283 mg (1 mMol) 4-(2,6-Dichloranilino)-3-thiophenacetonitril in 20 ml Methylenchlorid tropft man bei –15° C eine Lösung von 135 mg Sulfurylchlorid in 5 ml Methylenchlorid. Nach 10 Minuten versetzt man mit einer Natriumdithionitlösung und 3 ml 2 n-Natronlauge. Man trennt die organische Phase ab, trocknet, klärt mit Bleicherde (z. B. Tonsil[R]) und engt ein. Umkristallisation aus Methanol liefert 5-Chlor-4-(2,6-dichloranilino)-3-thiophenacetonitril (F. 128–128,5° C). Diese Verbindung dient als Vorprodukt für die nach Beispiel 2 erhältliche Verbindung.

Analog erhält man aus 4-(2-Chlor-6-methylanilino)-3-thiophenessigsäuremethylester durch Chlorieren mit Sulfurylchlorid 5-Chlor-4-(2-chlor-6-methylanilino)-3-thiophenessigsäuremethylester.

Beispiel 13

Zu einer Lösung von 3,02 g (10 mMol) 4-(2,6-Dichloranilino)-3-thiophenessigsäure in 15 ml Methylenchlorid tropft man bei –15 C 1,35 g (10 mMol) Sulfurylchlorid in 20 ml Methylenchlorid. Nach 10 Minuten versetzt man mit je 10 ml 2 n-NaOH und 10%iger Natriumdithionitlösung. Man trennt die wässerige Phase ab, säuert an, extrahiert mit Essigester, trocknet und engt ein. Umkristallisation aus Cyclohexan liefert reine 5-Chlor-4-(2,6-Dichloranilino)-3-thiophenessigsäure (Z. 141–142° C).

Analog erhält man aus 4-(2-Chlor-6-methylanilino)-3-thiophenessigsäure durch Chlorieren mit Sulfurylchlorid 5-Chlor-4-(2-chlor-6-methylanilino)-3-thiophenessigsäure.

Beispiel 14

2,84 g (10 mMol) N-(2,6 Dichlorphenyl)-2,3-dihydro-2-oxo-thieno[3,4-b]-pyrrol werden in 40 ml Methanol und 10 ml 6 n-KOH eine halbe Stunde gekocht. Man verdünnt mit 200 ml Wasser, extrahiert mit Toluol, säuert die wässerige Phase an und saugt den entstandenen Niederschlag von 4-(2,6-Dichloranilino)-3-thiophenessigsäure ab. Der Niederschlag wird mit Diisopropylether ausgekocht, getrocknet und schmilzt danach bei 179–181° C.

Die Ausgangsverbindung erhalt man auf folgende Weise:

1 g (0,33 mMol) 4-(2,6 Dichloranilino)-3-thiophenessigsäure und 0,7 g (0,33 mMol) Phosphorpentachlorid werden in 10 ml Toluol eine halbe Stunde bei Raumtemperatur gerührt. Man extrahiert mit Wasser und Natriumbicarbonatlosung, klärt mit Bleicherde (z. B. Tonsil[R]) und engt ein. Um-

kristallisation aus Isopropanol liefert N-(2,6-Dichlorphenyl)-2,3-dihydro-2-oxo-thieno[3,4-b]pyrrol (F. 187–188°C).

### Beispiel 15

2,83 g 4-(2,6-Dichloranilino)-3-thiophenacetonitril werden in 40 ml Diethylenglykol, die bei 0°C mit Salzsäuregas gesättigt wurden, eingetragen. Man rührt eine Stunde bei Raumtemperatur, gießt auf Wasser, extrahiert die wässerige Phase mit Essigester, wäscht mehrmals mit Wasser, trocknet und engt ein. Umkristallisation aus Cyclohexan liefert reinen 4-(2,6-Dichloranilino)-3-thiophenessigsäure-2-(2-hydroxyethoxy)-ethylester (F. 58°C).

### Beispiel 16

5,28 g (17,5 mMol) 4-(2,6-Dichloranilino)-3-thiophenessigsäure werden zusammen mit 2,6 g (17,5 mMol) Triethanolamin in 30 ml Chloroform gelöst, die Lösung wird mit 20 ml Wasser ausgeschüttelt. Nach Abdampfen des Wassers wird mit Ethanol aufgenommen und in einer Kristallisierschale durch Eindunsten zur Kristallisation gebracht. Man erhält das Triethanolammonium-4-(2,6-dichloranilino)-3-thiophenacetat.

Analog erhält man Piperazinium-bis[4-(2,6-dichloranilino)-3-thiophenacetat].

### Beispiel 17

30,2 g (0,1 Mol) 4-(2,6-Dichloranilino)-3-thiophenessigsäure werden in der Hitze in 100 ml einer 1 n-Natronlauge gelöst. Man läßt erkalten und filtriert das ausgefallene Natrium-4-(2,6-dichloranilino)-3-thiophenacetat ab (F. 247–250°C).

Analog erhält man das Natrium-3-(2,6-dichloranilino)-2-thiophenacetat (F. 229–232°C).

### Beispiel 18

10 000 Tabletten mit einem Wirkstoffgehalt von 30 mg werden wie nachfolgend beschrieben hergestellt:

300 g 4-(2,6-Dichlor-anilino)-3-thiophenessigsäure, 800 g Maisstärke, 550 g Milchzucker, 30 g amorphe Kieselsäure und 40 g Natriumlaurylsulfat werden gemischt und gesiebt. Diese Mischung wird mit einer Lösung von 50 g Polyvinylpyrrolidon in 320 ml Ethanol befeuchtet und durch ein Sieb mit einer Maschenweite von 1,25 mm granuliert. Das Granulat wird bei 40°C getrocknet und mit 160 g Pektin, 50 g Talk und 20 g Magnesiumstearat gemischt. Diese Mischung wird zu Tabletten à 200 mg mit 8 mm Durchmesser verpreßt.

### Beispiel 19

10 000 Kapseln mit einem Wirkstoffgehalt von 25 mg werden wie nachfolgend beschrieben hergestellt:

250 g 4-(2,6-Dichloranilino)-3-thiophenessigsäure, 745 g mikrokristalline Cellulose und 5 g ungepreßte amorphe Kieselsäure werden gut gemischt und in Hartgelatinekapseln Größe 4 abgefüllt.

### Beispiel 20

Herstellung einer Charge von 1000 Suppositorien

2,450 kg Suppositorienmasse (Suppocire® BM) werden auf 40 bis 45°C erhitzt. In die Schmelze werden 0,050 kg 4-(2,6-Dichloranilino)-3-thiophenessigsäure (als Natriumsalz) eingerührt.

Die Zäpfchenmasse wird homogenisiert und anschließend in Formen gegossen.

Beispiel 21

Herstellung einer Charge von 100 Liter Suspension

2,70 kg Tylose[R] C 30 werden unter kräftigem Rühren in 90 l Wasser gegeben; anschließend werden 1,00 kg 4-(2,6-Dichloranilino)-3-thiophenessigsäure, 0,11 kg Natriumcyclamat und 0,08 kg Sorbinsäure zugegeben. Mit Wasser wird auf 100 l ergänzt. Die Mischung wird durch eine Korundscheibenmühle geschickt, anschließend entlüftet und dann in 5 ml Fraktionen abgefüllt.

Beispiel 22

Herstellung von 100 Liter einer Injektionslösung

65 l destilliertes Wasser werden unter $N_2$-Begasung auf 80°C erhitzt, dann werden 2,000 kg 4-(2,6-Dichloranilino)-3-thiophenessigsäure (als Natriumsalz) und 0,150 kg Prednisolon zugegeben. Nach vollständigem Lösen wird auf Raumtemperatur abgekühlt, mit 0,200 kg Natriumdisulfit, 0,025 kg Cysteinhydrochlorid und 26,00 kg 1,2-Propylenglykol versetzt, mit destilliertem Wasser auf 100 l ergänzt und bis zur Lösung gerührt.

Pharmakologie

Die ausgeprägte antiphlogistische, analgetische und antipyretische Wirkung der erfindungsgemäßen Verbindungen läßt sich in verschiedenen Tests demonstrieren. Als Beispiel werden die erfindungsgemäßen Verbindungen im Vergleich mit der handelsüblichen Verbindung 2 bzw. mit der in der Arzneim.-Forsch. (Drug Res.) 20 (1970) 293—94 beschriebenen Verbindung 1 untersucht. Die folgende Tabelle 1 gibt den Zusammenhang zwischen laufender Nr. und Name der untersuchten Verbindung wieder.

Tabelle 1

| lfd. Nr. | Name der Verbindung |
|---|---|
| 1 | 3-(2,6-Dichloranilino)-thiophen-4-carbonsäure |
| 2 | Indometacin |
| 3 | 4-(2,6-Dichloranilino)-3-thiophenessigsäure |
| 4 | 4-(2-Chlor-6-methylanilino)-3-thiophenessigsäure |
| 5 | 4-(2,6-Dichlor-3-methylanilino)-3-thiophenessigsäure |
| 6 | 3-(2,6-Dichloranilino)-2-thiophenessigsäure |
| 7 | 4-(2-Chlor-3-methylanilino)-3-thiophenessigsäure |
| 8 | 4-(3-Chlor-2-methylanilino)-3-thiophenessigsäure |
| 9 | 4-(2,6-Dimethylanilino)-3-thiophenessigsäure |
| 10 | 5-Chlor-4-(2,6-dichloranilino)-3-thiophenessigsäure |
| 11 | 3-(2-Chlor-6-methylanilino)-2-thiophenessigsäure |
| 12 | 4-(2,6-Difluoranilino)-3-thiophenessigsäure |

Tabelle 2 gibt die akute antiphlogistische Wirkung, die Toxizität und den therapeutischen Quotienten der untersuchten Verbindungen wieder.

Tabelle 2

Antiphlogistische Wirkung der erfindungsgemäßen Verbindungen im akuten Entzündungsmodell – gemessen am Einfluß auf das Carrageninödem der Rattenhinterpfote – nach einmaliger oraler Verabreichung sowie letale Wirkung bei der Ratte

| Verbindung | Dosis | Max. Hemmung des Carrageninödems innerh. 7 Std. nach oraler Substanzgabe | | Therapeutischer Quotient | Letale Wirkung nach 7tägiger Verabreichung (Ratte) $LD_{50}$ |
|---|---|---|---|---|---|
| | | | $ED_{50}$* | $LD_{50}/ED_{50}$ | |
| | mg/kg p. o. | % | mg/kg p. o. | | mg/kg/die p. o. |
| 1 | 10.0 | 31 | 56 | 2.1 | 120 |
| | 100.0 | 57 | | | |
| | 1.0 | 29 | | | |
| 2 | 3.0 | 47 | 4.6 | 1.2 | 5.5 |
| | 10.0 | 56 | | | |
| | 0.3 | 29 | | | |
| 3 | 1.0 | 46 | 1.6 | 6.9 | 11 |
| | 3.0 | 56 | | | |
| 4 | 3.0 | 50 | 3 | ≥6.7 | ≥20 |
| 6 | 3.0 | 52 | ~3 | ≥6.7 | ≥20 |
| 8 | 3.0 | 49 | ~3 | ≥10 | ≥30 |
| 11 | 3.0 | 49 | ~3 | ≥6.7 | ≥20 |

*) $ED_{50}$ = Dosis, die eine maximale Ödemhemmung um 50% bewirkt.

Der Einfluß der zu testenden Verbindungen auf das Carrageeninödem der Rattenhinterpfote wurde wie folgt bestimmt:

Weibliche Sprague-Dawley-Ratten (Gruppen zu je 10 Tieren; Gewicht je Tier 140–170 g), denen ca. 16 Stunden zuvor das Futter (Altromin[R] R, Wasser ad libitum) entzogen wird, erhalten 1 Stunde nach oraler Gabe der zu prüfenden Verbindungen je Tier 0,05 ml einer 1%igen Carrageeninsuspension subplantar in die rechte Hinterpfote injiziert. Die Ratten werden bei 24° C gehalten.

Die Bestimmung des Pfotenvolumens jeder Ratte (jeweils 2–3 Einzelmessungen) erfolgt vor sowie in stündlichen Abständen während 7 Stunden nach Ödemprovokation. Die zu den genannten Zeiten nach der Carrageenin-Injektion ermittelte durchschnittliche prozentuale Pfotenschwellung jeder behandelten Gruppe wird auf diejenige der unbehandelten Kontrollgruppe (=100%) bezogen.

Als Maß für die antiphlogistische Wirkung dient die maximale prozentuale Ödemhemmung.

Die Bestimmung des Pfotenvolumens erfolgt plethysmometrisch. Meßprinzip: Das durch die Rattenpfote verdrängte Flüssigkeitsvolumen wird über einen elektromechanischen Druckwandler (Statham P 23 V, 0–200 mm Hg) digital registriert.

Die angegebenen Dosen der geprüften Verbindungen wurden in einem Flüssigkeitsvolumen von 20 ml/kg verabreicht.

Tabelle 3 und Tabelle 4 geben die chronische antiphlogistische Wirkung, die Toxizität und den therapeutischen Quotienten der untersuchten Verbindungen wieder.

Tabelle 3

Antiphlogistische Wirkung der erfindungsgemäßen Verbindung im chronischen Entzündungsmodell — gemessen am Einfluß auf die Granulationsgewebsbildung nach sc. Watte-Implantation (sog. Cotton-pellet-Test) — sowie letale Wirkung bei der Ratte nach 7tägiger oraler Verabreichung

| Verbindung | Dosis 7 × mg/kg/die oral | Hemmung der Granulationsgewebsbildung nach 7tägiger Behandlung % | $ED_{20}$*) mg/kg/die p. o. | Therapeut. Quotient $LD_{50}/ED_{20}$ | Letale Wirkung nach 7tägiger oraler Verabreichung (Ratte) $LD_{50}$ mg/kg/die |
|---|---|---|---|---|---|
| | 0.1 | 7 | | | |
| | 0.3 | 11 | | | |
| 2 | 1.0 | 16 | ~3 | ~1.8 | 5.5 |
| | 3.0 | 16 | | | |
| | 4.0 | 17 | | | |
| | 5.0 | (25% tot) | | | |
| | 0.1 | 6 | | | |
| | 0.3 | 15 | | | |
| 3 | 1.0 | 14 | ~1 | ~11 | 11 |
| | 3.0 | 22 | | | |
| | 5.0 | 22 | | | |
| | 6.0 | 36 (0% tot) | | | |
| 5 | 1.0 | 16 | 1.6 | 5 | 8 |
| | 3.0 | 26 | | | |
| 7 | 3.0 | 19 | ~3.5 | ≥5.7 | ≥20 |
| | 10.0 | 27 | | | |
| 9 | 3.0 | 19 | ~3.0 | ≥10 | ≥30 |

*) $ED_{20}$ = Dosis, die eine durchschnittliche Hemmung der Granulationsgewebsbildung um 20% bewirkt.

Versuchsdurchführung zur Untersuchung des Einflusses der zu testenden Verbindungen auf die Granulationsgewebsbildung nach Watte-Implantation bei der Ratte (sog. Cotton-pellet-Methode):

Männliche Sprague-Dawley-Ratten (jeweils Gruppen zu 8 Tieren; Gewicht je Tier 150—170 g) erhalten in Diethylethernarkose und unter sterilen Bedingungen im Schulterblattbereich beidseitig je 1 Wattekügelchen (Hersteller: Firma Hartmann/Heidenheim; Wattekügelchen Gr. 2, Nr. 4865/2) von 13,0 ± 0,5 mg subcutan implantiert, das zuvor mit 0,1 ml einer Lösung von 0,5 mg Penicillin-G und 0,8 mg Streptomycinsulfat/1 ml aqua dest. getränkt wurde. Der Verschluß des Hautschnitts erfolgt mittels Klammern.

An sieben aufeinanderfolgenden Tagen werden die zu prüfenden Verbindungen (als Natrium-Salz in wässeriger Lösung) bzw. die entsprechende Menge (5 ml/kg/die) Leitungswasser (= Kontrollgruppe) täglich oral verabreicht. Am achten Tag werden die Tiere getötet, die Wattegranulate vorsichtig, d. h. unter Schonung der Bindegewebskapsel, freipräpariert, getrocknet (15 Stunden bei 120°C) und gewogen. Durch Abzug des Gewichtsanteils der Wattekügelchen erhält man die Menge des neugebildeten Granulationsgewebes.

Als Maß für die antiproliferative Wirkung einer Verbindung dient die prozentuale Minderung des mittleren Granulom-Trockengewichtes einer behandelten Gruppe gegenüber der Kontrollgruppe (= 100%).

Tabelle 4
Antiarthritische Wirkung (sog. Adjuvans-Arthritis; Ratte; $ED_{25}$; 5tägige orale Verabreichung) und Toxizität ($LD_{50}$ nach 7tägiger oraler Applikation bei der Ratte)

| Substanz; lfd. Nr. | Hemmung der Adjuvans-Arthritis um 25% (= $ED_{25}$) bei der Ratte nach 5tägiger oraler Applikation: $ED_{25}$ mg/kg/die p. o. | Therapeutischer Quotient $LD_{50}/ED_{25}$ | $LD_{50}$ nach 7tägiger Verabreichung Ratte mg/kg/die oral |
|---|---|---|---|
| 1 | 9 | 13.3 | 120 |
| 2 | 0.18 | 30.6 | 5.5 |
| 3 | 0.20 | 55 | 11 |

Der Einfluß der zu prüfenden Verbindungen auf die durch Mycobacterium butyricum hervorgerufene Polyarthritis der Ratte (sog. »Adjuvans-Arthritis«) wurde mit der nachfolgend beschriebenen Versuchsanordnung bestimmt: In Anlehnung an die von NEWBOULD and PERRINE et al. [NEWBOULD, B. B.; Brit. J. Pharmacol. 21, 127 (1963); PERRINE, J. W. u. TAKESUE, E. I.; Arch. int. Pharmacodyn. 174, 192–198 (1968)] beschriebene Methodik wird an Ratten durch intradermale Injektion einer Myobact. butyricum Paraffinöl-Suspension in die Schwanzwurzel eine sog. Adjuvans-Arthritis ausgelöst, die sich vornehmlich in einem starken Anschwellen des Schwanzes (Primärläsion) und der Pfoten (Sekundärläsion) äußert. Antiphlogistika hemmen diese Schwellungsreaktion.
In Tabelle 5 und 6 ist die analgetische (Entzündungsschmerz) und antipyretische (Hefe-Fieber) Wirkung der geprüften Verbindungen nebst letaler Wirkung und therapeutischem Quotienten aufgeführt.

Tabelle 5
Analgetische Wirkung der erfindungsgemäßen Verbindungen nach einmaliger oraler Verabreichung — gemessen am Einfluß auf die durch Beugen eines entzündeten Fußgelenkes ($AgNO_3$-Arthritis; Entzündungsschmerz) bei der Ratte provozierte Schmerzreaktion (Vokalisation) — sowie Toxizität nach 7tägiger Verabreichung bei der Ratte

| Verb. | Dosis mg/kg p. o. | Analgetische Wirkung Hemmung der Schmerzreaktion um % | $ED_{25}$*) mg/kg | $ED_{50}$*) p. o. | Therapeut. quotienten $\frac{LD_{50}}{ED_{25}}$ | $\frac{LD_{50}}{ED_{50}}$ | Letale Wirkung nach 7tägiger oraler Applikat. (Ratte) $LD_{50}$ mg/kg/die |
|---|---|---|---|---|---|---|---|
| | 3 | 10 | | | | | |
| 1 | 20 | 20 | 30 | 140 | 4 | 0.86 | 120 |
| | 100 | 40 | | | | | |
| | 200 | 60 | | | | | |
| | 1 | 23 | | | | | |
| 2 | 3 | 36 | 1.2 | 6 | 4.58 | 0.92 | 5.5 |
| | 6 | 50 | | | | | |
| | 1 | 26 | | | | | |
| 3 | 3 | 50 | 1 | 3 | 11 | 3.67 | 11 |
| | 10 | 62 | | | | | |

*) $ED_{25}$, $ED_{50}$ = Dosis, die zum Zeitpunkt des Wirkungsmaximums bei 25 bzw. 50% der Tiere die Schmerzreaktion vollständig unterdrückt.

Der Einfluß der zu prüfenden Verbindungen auf die durch Beugen eines entzündeten Tarsalgelenkes ($AgNO_3$-Entzündung) bei der Ratte provozierte Schmerzreaktion wurde wie folgt bestimmt:
In Anlehnung an HOFFMEISTER et al. [Arzneim. Forsch. 24, (1974) 600] erhalten weibliche Sprague-Dawley-Ratten (je Tier ca. 160–180 g) unter Ethernarkose 0,2 ml einer 1%igen Silbernitratlösung in das rechte Hinterpfoten-Sprunggelenk (Innenseite) injiziert. Die Tiere werden bei 24°C gehalten und erhalten als Futter Altromin[R] R und Wasser ad libitum. Nach der $AgNO_3$-Injektion läßt sich bei nahezu allen Tieren (90–95%) durch mäßiges Beugen des Sprunggelenkes eine

Schreireaktion auslösen. Alle positiv reagierenden Tiere werden in Gruppen zu 10 Tieren zusammengefaßt. Die zu prüfenden Verbindungen werden unmittelbar nach der $AgNO_3$-Applikation in einem Flüssigkeitsvolumen von 10 ml/kg oral verabreicht; die unbehandelten Kontrolltiere erhalten die entsprechende Menge Leitungswasser.

Innerhalb 4 Stunden nach Substanzgabe wird in halbstündlichen Abständen die Zahl der reagierenden Tiere ermittelt.

Als Maß für die analgetische Wirkung dient der maximale %-Anteil der geschützten Tiere innerhalb 4 Stunden nach Substanzgabe [bezogen auf die Anzahl der reagierenden Tiere der Kontrollgruppe (= 100%)].

Tabelle 6
Antipyretische Wirkung der erfindungsgemäßen Verbindungen — gemessen am Einfluß auf das Hefe-Fieber der Ratte — nach einmaliger oraler Gabe sowie letale Wirkung

| Verb. | Dosis<br>mg/kg p. o. | Maximale Senkung des Hefefiebers während 7 Std. nach Substanzgabe<br>um 0°C | $ED_{1.5}$*)<br>mg/kg p. o. | Therapeut. Quotient<br>$LD_{50}/ED_{1.5}$ | Letale Wirkung nach 7tägiger Verabreichung (Ratte)<br>$LD_{50}$<br>mg/kg/die |
|---|---|---|---|---|---|
| | 1 | 0 | | | |
| 1 | 3 | 0.6 | 8.2 | 14.6 | 120 |
| | 10 | 1.7 | | | |
| | 30 | 2.1 | | | |
| | 1 | 0.2 | | | |
| 2 | 3 | 0.7 | 5.0 | 1.1 | 5.5 |
| | 6 | 1.8 | | | |
| | 0.03 | 0.4 | | | |
| 3 | 0.1 | 0.8 | 0.4 | 27.5 | 11 |
| | 0.3 | 1.4 | | | |
| | 1.0 | 1.7 | | | |
| 4 | 0.3 | 1.4 | ~0.3 | ≫66.7 | ≫20 |
| 5 | 0.3 | 1.7 | <0.3 | >26.7 | 8 |
| 6 | 0.3 | 1.7 | <0.3 | ≫66.7 | ≫20 |
| | 0.1 | 1.0 | | | |
| 9 | 0.3 | 1.5 | 0.3 | >100 | >30 |
| | 1.0 | 2.3 | | | |
| 10 | 0.3 | 1.2 | 0.5 | ≫40 | ≫20 |
| | 0.6 | 1.6 | | | |
| 12 | 0.3 | 0.5 | 0.9 | >33 | >30 |
| | 1.0 | 1.6 | | | |

*) $ED_{1.5}$ = Dosis, die eine maximale Senkung des Hefe-Fiebers um 1.5°C bewirkt.

Die antipyretische Wirkung wird am Einfluß der zu prüfenden Verbindungen auf das Hefefieber der Ratte ermittelt.

Bei weiblichen Sprague-Dawley-Ratten (in Gruppen zu 5 Tieren; Tiergewicht 160—180 g) wird durch subcutane Injektion (Nacken) von 10 ml/kg einer 20%igen wässerigen Bierhefe-Suspension (Bierhefe »Heliosan«/Hefereformwerk Radolfzell) eine Hyperthermie erzeugt. 24 Stunden nach der Hefe-Injektion erhalten die Tiere die zu prüfenden Verbindungen oral in einem Flüssigkeitsvolumen von 5 ml/kg verabreicht, eine Kontrollgruppe erhält die entsprechende Menge Leitungswasser. 18 Stunden zuvor wurde den Ratten das Futter (Altromin® R, Wasser ad libitum) entzogen. Die Ratten werden bei ca. 23°C gehalten.

Die Körpertemperatur wird 1 Stunde vor und in stündlichen Abständen nach Substanzgabe registriert.

Als Maß für die antipyretische Wirkung dient die maximale Temperatursenkung (bezogen auf die Temperatur vor Substanzgabe) im Verlauf von 6 Stunden. Die Körpertemperatur wird mit einem Temperaturfühler (Tastomed H, Braun electronic) gemessen.

Aus den vorstehenden Daten der Tabellen 2—6 ergibt sich die Überlegenheit der erfindungsgemäßen Verbindungen über die des Standes der Technik.

**Patentansprüche für die benannten Vertragsstaaten: CH, DE, IT, LU, NL, SE**

1. Phenylaminothiophenessigsäuren der allgemeinen Formel I

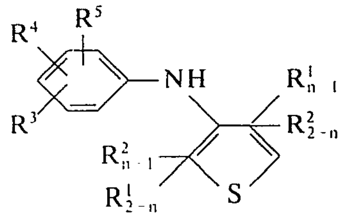

(I)

worin

$R^1$ ein Wasserstoffatom, ein Chloratom, ein Bromatom oder eine Methylgruppe,
$R^2$ eine $-CH_2-COOH$-Gruppe oder eine $-CH_2-COOR^6$-Gruppe und
$R^3$ ein Wasserstoffatom, ein Halogenatom, eine Alkylgruppe, eine Alkoxygruppe oder eine Trifluormethylgruppe bedeuten,
$R^4$ eine der Bedeutungen von $R^3$ hat,
$R^5$ ein Wasserstoffatom, ein Halogenatom oder eine Alkylgruppe,
$R^6$ eine gegebenenfalls durch Hydroxy-, Hydroxyalkoxy- oder Alkanoyloxy-Gruppen substituierte Alkylgruppe mit 1 bis 5 Kohlenstoffatomen oder eine Benzylgruppe und
n 1 oder 2 bedeuten,

sowie die Salze der Säuren.

2. Phenylaminothiophenessigsäuren der allgemeinen Formel I*

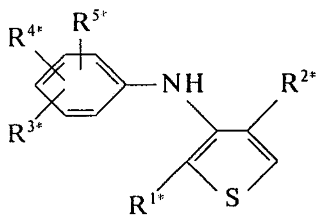

(I*)

worin

$R^{1*}$ ein Wasserstoffatom, ein Chloratom, ein Bromatom oder eine Methylgruppe,
$R^{2*}$ eine $-CH_2-COOH$-Gruppe oder eine $-CH_2-COOR^{6*}$-Gruppe und
$R^{3*}$ ein Wasserstoffatom, ein Chloratom, eine Methylgruppe oder eine Trifluormethylgruppe bedeuten,
$R^{4*}$ eine der Bedeutungen von $R^{3*}$ hat,
$R^{5*}$ ein Wasserstoffatom oder ein Chloratom und
$R^{6*}$ eine gegebenenfalls durch eine Acetoxygruppe oder eine 2-Hydroxyethoxygruppe substituierte Alkylgruppe mit 1 bis 4 Kohlenstoffatomen oder eine Benzylgruppe bedeuten,

sowie die Salze der Säuren.

3. Verbindungen nach Anspruch 2, in denen $R^{1*}$ ein Wasserstoffatom oder ein Chloratom, $R^{2*}$ eine $-CH_2-COOH$-Gruppe oder eine $-CH_2-COOR^{6*}$-Gruppe, $R^{3*}$ ein Chloratom, eine Methylgruppe oder eine Trifluormethylgruppe, $R^{4*}$ ein Chloratom oder eine Methylgruppe, $R^{5*}$ ein Wasserstoffatom oder ein Chloratom und $R^{6*}$ eine 2-(2-Hydroxyethoxy)-ethylgruppe bedeuten, sowie die pharmakologisch verträglichen Salze der Säuren.

4. Verbindungen nach Anspruch 2, in denen $R^{1*}$ ein Wasserstoffatom, $R^{2*}$ eine $-CH_2-COOH$-Gruppe, $R^{3*}$ ein Chloratom oder eine Methylgruppe, $R^{4*}$ ein Chloratom und $R^{5*}$ ein Wasserstoffatom bedeuten, sowie ihre pharmakologisch verträglichen Salze.

5. Phenylaminothiophenessigsäuren der allgemeinen Formel I**

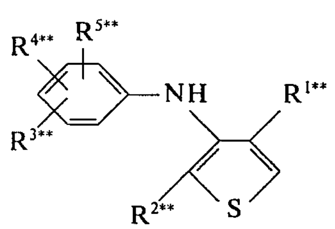

(I**)

worin

$R^{1**}$ ein Wasserstoffatom oder eine Methylgruppe,
$R^{2**}$ eine $-CH_2-COOH$-Gruppe oder eine $-CH_2-COOR^{6**}$-Gruppe und
$R^{3**}$ ein Wasserstoffatom, ein Chloratom, eine Methylgruppe oder eine Trifluormethylgruppe bedeuten,
$R^{4**}$ eine der Bedeutungen von $R^{3**}$ hat,
$R^{5**}$ ein Wasserstoffatom oder ein Chloratom und
$R^{6**}$ eine gegebenenfalls durch eine Acetoxygruppe oder eine 2-Hydroxyethoxygruppe substituierte Alkylgruppe mit 1 bis 4 Kohlenstoffatomen oder eine Benzylgruppe bedeuten,

sowie die Salze der Säuren.

6. Verbindungen nach Anspruch 5, in denen $R^{1**}$ ein Wasserstoffatom, $R^{2**}$ eine $-CH_2-COOH$-Gruppe oder eine $-CH_2-COOR^{6**}$-Gruppe, $R^{3**}$ ein Chloratom, eine Methylgruppe oder eine Trifluormethylgruppe, $R^{4**}$ ein Chloratom oder eine Methylgruppe, $R^{5**}$ ein Wasserstoffatom oder ein Chloratom und $R^{6**}$ eine 2-(2-Hydroxyethoxy)-ethylgruppe bedeuten, sowie die pharmakologisch verträglichen Salze der Säuren.

7. Verbindungen nach Anspruch 5, in denen $R^{1**}$ ein Wasserstoffatom, $R^{2**}$ eine $-CH_2-COOH$-Gruppe, $R^{3**}$ ein Chloratom oder eine Methylgruppe, $R^{4**}$ ein Chloratom und $R^{5**}$ ein Wasserstoffatom bedeuten, sowie ihre pharmakologisch verträglichen Salze.

8. 4-(2,6-Dichlor-anilino)-3-thiophenessigsäure und ihre pharmakologisch verträglichen Salze.

9. Arzneimittel, enthaltend eine oder mehrere Verbindungen gemäß Anspruch 1 bis 8.

10. Verfahren zur Herstellung von Phenylaminothiophenessigsäuren und deren Salzen gemäß Anspruch 1, dadurch gekennzeichnet, daß man funktionelle Carbonsäurederivate der allgemeinen Formel II

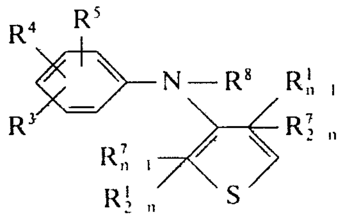

(II)

worin $R^1$, $R^3$, $R^4$, $R^5$ und n die im Anspruch 1 angegebenen Bedeutungen haben, $R^7$ eine $-CH_2-G$-Gruppe oder gemeinsam mit $R^8$ eine $-CH_2-CO$-Gruppe, $R^8$ ein Wasserstoffatom oder gemeinsam mit $R^7$ eine $-CO-CH_2$-Gruppe und G ein funktionelles Derivat einer Carbonsäuregruppe darstellt, lyolysiert und gegebenenfalls anschließend die erhaltenen Säuren bzw. Ester der allgemeinen Formel I in üblicher Weise ineinander und/oder in die Salze überführt.

11. Verfahren zur Herstellung von Phenylaminothiophenessigsäuren und deren Salzen gemäß Anspruch 2, dadurch gekennzeichnet, daß man funktionelle Phenylaminothiophenessigsäurederivate der allgemeinen Formel II*

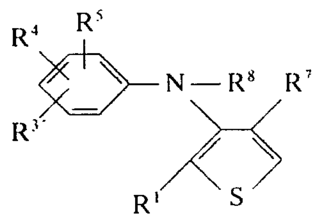

(II*)

worin $R^{1*}$, $R^{3*}$, $R^{4*}$ und $R^{5*}$ die im Anspruch 2 angegebenen Bedeutungen haben, $R^{7*}$ eine $-CH_2-G^*$-Gruppe oder gemeinsam mit $R^{8*}$ eine $-CH_2-CO$-Gruppe, $R^{8*}$ ein Wasserstoffatom oder gemeinsam mit $R^{7*}$ eine $-CO-CH_2$-Gruppe und $G^*$ ein funktionelles Derivat einer Carbonsäuregruppe darstellt, lyolysiert und gegebenenfalls anschließend die erhaltenen Säuren bzw. Ester der allgemeinen Formel I* halogeniert und/oder in üblicher Weise ineinander und/oder in die Salze überführt.

12. Verfahren zur Herstellung von Phenylaminothiophenessigsäuren und deren Salzen gemäß Anspruch 5, dadurch gekennzeichnet, daß man

a) funktionelle Phenylaminothiophenessigsäurederivate der allgemeinen Formel II**

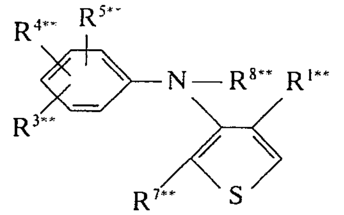

(II**)

worin $R^{1**}$, $R^{3**}$, $R^{4**}$ und $R^{5**}$ die im Anspruch 5 angegebenen Bedeutungen haben, $R^{7**}$ eine $-CH_2-G^{**}$-Gruppe oder gemeinsam mit $R^{8**}$ eine $-CH_2-CO$-Gruppe, $R^{8**}$ ein Wasserstoffatom oder gemeinsam mit $R^{7**}$ eine $-CO-CH_2$-Gruppe und $G^{**}$ ein funktionelles Derivat einer Carbonsäuregruppe darstellt, lyolysiert und gegebenenfalls anschließend die erhaltenen Säuren bzw. Ester der allgemeinen Formel I** in üblicher Weise ineinander und/oder in die Salze überführt oder

b) ein Phenylaminothiophenessigsäurederivat der allgemeinen Formel III**

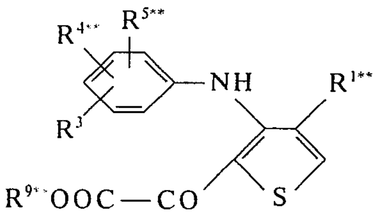

(III**)

worin $R^{1**}$, $R^{3**}$, $R^{4**}$ und $R^{5**}$ die im Anspruch 5 angegebenen Bedeutungen haben und $R^{9**}$ ein Wasserstoffatom oder eine Alkylgruppe mit 1 bis 5 Kohlenstoffatomen darstellt, oder ein Salz davon reduziert und gegebenenfalls anschließend die erhaltenen Säuren bzw. Ester der allgemeinen Formel I** in üblicher Weise ineinander und/oder in die Salze überführt.

13. Wirkstoffe nach Anspruch 1 bis 8 zur Verwendung bei der Bekämpfung von Krankheiten, die mit Entzündungen, Schmerzen oder Fieber verbunden sind.

14. Verwendung von Wirkstoffen nach Anspruch 1 bis 8 zur Herstellung von Arzneimitteln, die zur Bekämpfung von Krankheiten, die mit Entzündungen, Schmerzen oder Fieber verbunden sind, eingesetzt werden.

**Patentansprüche für den Vertragsstaat: AT**

1. Verfahren zur Herstellung von Phenylaminothiophenessigsäuren der allgemeinen Formel I

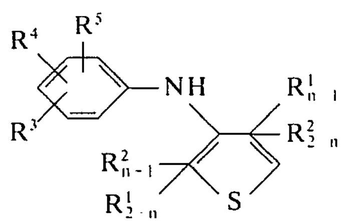

(I)

worin

- $R^1$ ein Wasserstoffatom, ein Chloratom, ein Bromatom oder eine Methylgruppe,
- $R^2$ eine $-CH_2-COOH$-Gruppe oder eine $-CH_2-COOR^6$-Gruppe und
- $R^3$ ein Wasserstoffatom, ein Halogenatom, eine Alkylgruppe, eine Alkoxygruppe oder eine Trifluormethylgruppe bedeuten,
- $R^4$ eine der Bedeutungen von $R^3$ hat,
- $R^5$ ein Wasserstoffatom, ein Halogenatom oder eine Alkylgruppe,
- $R^6$ eine gegebenenfalls durch Hydroxy-, Hydroxyalkoxy- oder Alkanoyloxygruppen substituierte Alkylgruppe mit 1 bis 5 Kohlenstoffatomen oder eine Benzylgruppe und
- n 1 oder 2 bedeuten,

und den Salzen der Säuren, dadurch gekennzeichnet, daß man funktionelle Carbonsäurederivate der allgemeinen Formel II

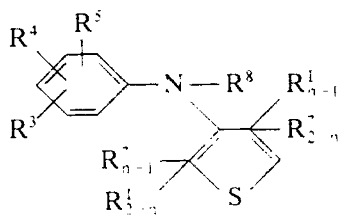

(II)

worin $R^1$, $R^3$, $R^4$, $R^5$ und n die oben angegebenen Bedeutungen haben, $R^7$ eine $-CH_2-G$-Gruppe oder gemeinsam mit $R^8$ eine $-CH_2-CO$-Gruppe, $R^8$ ein Wasserstoffatom oder gemeinsam mit $R^7$ eine $-CO-CH_2$-Gruppe und G ein funktionelles Derivat einer Carbonsäuregruppe darstellt, lyolysiert und gegebenenfalls anschließend die erhaltenen Säuren bzw. Ester der allgemeinen Formel I in üblicher Weise ineinander und/oder in die Salze überführt.

2. Verfahren zur Herstellung von Phenylaminothiophenessigsäuren der allgemeinen Formel I*

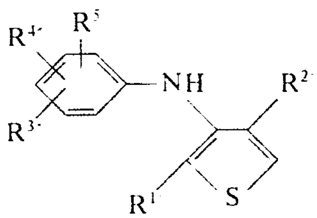

(I*)

worin

$R^{1*}$ ein Wasserstoffatom, ein Chloratom, ein Bromatom oder eine Methylgruppe,
$R^{2*}$ eine $-CH_2-COOH$-Gruppe oder eine $-CH_2-COOR^{6*}$-Gruppe und
$R^{3*}$ ein Wasserstoffatom, ein Chloratom, eine Methylgruppe oder eine Trifluormethylgruppe bedeuten,
$R^{4*}$ eine der Bedeutungen von $R^{3*}$ hat,
$R^{5*}$ ein Wasserstoffatom oder ein Chloratom und
$R^{6*}$ eine gegebenenfalls durch eine Acetoxygruppe oder eine 2-Hydroxyethoxygruppe substituierte Alkylgruppe mit 1 bis 4 Kohlenstoffatomen oder eine Benzylgruppe bedeuten,

und den Salzen der Säuren, dadurch gekennzeichnet, daß man funktionelle Phenylaminothiophenessigsäurederivate der allgemeinen Formel II*

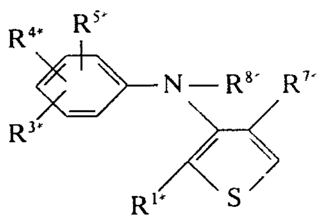

(II*)

worin $R^{1*}$, $R^{3*}$, $R^{4*}$ und $R^{5*}$ die oben angegebenen Bedeutungen haben, $R^{7*}$ eine $-CH_2-G^*$-Gruppe oder gemeinsam mit $R^{8*}$ eine $-CH_2-CO$-Gruppe, $R^{8*}$ ein Wasserstoffatom oder gemeinsam mit $R^{7*}$ eine $-CO-CH_2$-Gruppe und $G^*$ ein funktionelles Derivat einer Carbonsäuregruppe darstellt, lyolysiert und gegebenenfalls anschließend die erhaltenen Säuren bzw. Ester der allgemeinen Formel I* halogeniert und/oder in üblicher Weise ineinander und/oder in die Salze überführt.

3. Verfahren zur Herstellung von Phenylaminothiophenessigsäuren der allgemeinen Formel I**

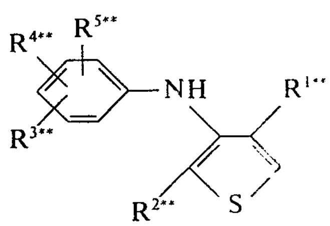

(I**)

worin

$R^{1**}$ ein Wasserstoffatom oder eine Methylgruppe,
$R^{2**}$ eine $-CH_2-COOH$-Gruppe oder eine $-CH_2-COOR^{6**}$-Gruppe und

$R^{3**}$ ein Wasserstoffatom, ein Chloratom, eine Methylgruppe oder eine Trifluormethylgruppe bedeuten,

$R^{4**}$ eine der Bedeutungen von $R^{3**}$ hat,

$R^{5**}$ ein Wasserstoffatom oder ein Chloratom und

$R^{6**}$ eine gegebenenfalls durch eine Acetoxygruppe oder eine 2-Hydroxyethoxygruppe substituierte Alkylgruppe mit 1 bis 4 Kohlenstoffatomen oder eine Benzylgruppe bedeuten,

und den Salzen der Säuren, dadurch gekennzeichnet, daß man

a) funktionelle Phenylaminothiophenessigsäurederivate der allgemeinen Formel II**

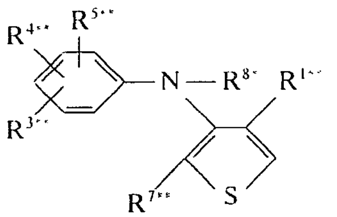

(II**)

worin $R^{1**}$, $R^{3**}$, $R^{4**}$ und $R^{5**}$ die oben angegebenen Bedeutungen haben, $R^{7**}$ eine $-CH_2-G^{**}$-Gruppe oder gemeinsam mit $R^{8**}$ eine $-CH_2-CO$-Gruppe, $R^{8**}$ ein Wasserstoffatom oder gemeinsam mit $R^{7**}$ eine $-CO-CH_2$-Gruppe und $G^{**}$ ein funktionelles Derivat einer Carbonsäuregruppe darstellt, lyolysiert und gegebenenfalls anschließend die erhaltenen Säuren bzw. Ester der allgemeinen Formel I** in üblicher Weise ineinander und/oder in die Salze überführt oder

b) ein Phenylaminothiophenessigsäurederivat der allgemeinen Formel III**

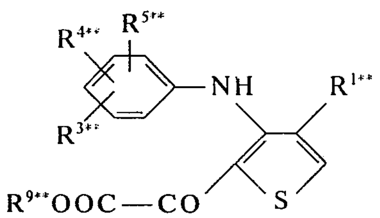

(III**)

worin $R^{1**}$, $R^{3**}$, $R^{4**}$ und $R^{5**}$ die oben angegebenen Bedeutungen haben und $R^{9**}$ ein Wasserstoffatom oder eine Alkylgruppe mit 1 bis 5 Kohlenstoffatomen darstellt, oder ein Salz davon reduziert und gegebenenfalls anschließend die erhaltenen Säuren bzw. Ester der allgemeinen Formel I** in üblicher Weise ineinander und/oder in die Salze überführt.

4. Verfahren nach Anspruch 1 oder 2 oder 3, dadurch gekennzeichnet, daß das funktionelle Carbonsäurederivat G bzw. G* bzw. G** eine Nitrilgruppe, eine Carbonsäurealkylestergruppe mit 1 bis 7 Kohlenstoffatomen im Alkylrest, eine Carbonsäurebenzylester- oder eine Carbonsäureamidgruppe darstellt.

5. Verfahren nach Anspruch 1 oder 2 oder 3, dadurch gekennzeichnet, daß die Lyolyse in Form der Solvolyse durchgeführt wird.

6. Verfahren nach Anspruch 2, dadurch gekennzeichnet, daß Verbindungen der allgemeinen Formel I*, worin $R^{1*}$ ein Wasserstoffatom oder ein Chloratom, $R^{2*}$ eine $-CH_2-COOH$-Gruppe oder eine $-CH_2-COOR^{6*}$-Gruppe, $R^{3*}$ ein Chloratom, eine Methylgruppe oder eine Trifluormethylgruppe, $R^{4*}$ ein Chloratom oder eine Methylgruppe, $R^{5*}$ ein Wasserstoffatom oder ein Chloratom und $R^{6*}$ eine 2-(2-Hydroxyethoxy)-ethylgruppe bedeuten, oder die pharmakologisch verträglichen Salze der Säuren hergestellt werden.

7. Verfahren nach Anspruch 2, dadurch gekennzeichnet, daß Verbindungen der allgemeinen Formel I*, worin $R^{1*}$ ein Wasserstoffatom, $R^{2*}$ eine $-CH_2-COOH$-Gruppe, $R^3$ ein Chloratom oder eine Methylgruppe, $R^{4*}$ ein Chloratom und $R^{5*}$ ein Wasserstoffatom bedeuten, oder ihre pharmakologisch verträglichen Salze hergestellt werden.

8. Verfahren nach Anspruch 2, dadurch gekennzeichnet, daß 4-(2,6-Dichloranilino)-3-thiophenessigsäure oder ihre pharmakologisch verträglichen Salze durch Hydrolyse von 4-(2,6-Dichloranilino)-3-thiophenacetonitril oder 4-(2,6-Dichloranilino)-3-thiophenessigsäurealkylestern und ggf. anschließende Überführung in die Salze hergestellt werden.

9. Verfahren nach Anspruch 3, dadurch gekennzeichnet, daß Verbindungen der allgemeinen Formel I**, worin $R^{1**}$ ein Wasserstoffatom, $R^{2**}$ eine $-CH_2-COOH$-Gruppe oder eine $-CH_2-COOR^{6**}$-Gruppe, $R^{3**}$ ein Chloratom, eine Methylgruppe oder eine Trifluormethylgruppe, $R^{4**}$ ein Chloratom oder eine Methylgruppe, $R^{5**}$ ein Wasserstoffatom oder ein Chloratom und $R^{6**}$ eine 2-(2-Hydroxyethoxy)-ethylgruppe bedeuten, oder die pharmakologisch verträglichen Salze der Säuren hergestellt werden.

10. Verfahren nach Anspruch 3, dadurch gekennzeichnet, daß Verbindungen der allgemeinen For-

mel I**, worin R1** ein Wasserstoffatom, R2** eine $-CH_2-COOH$-Gruppe, R3** ein Chloratom oder eine Methylgruppe, R4** ein Chloratom und R5** ein Wasserstoffatom bedeuten, oder ihre pharmakologisch verträglichen Salze hergestellt werden.

**Claims for the contracting States: CH, DE, IT, LU, NL, SE**

1. Phenylaminothiophenacetic acids of the general formula I

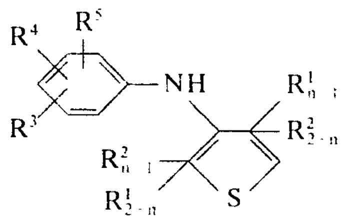

(I)

wherein

$R^1$ denotes a hydrogen atom, a chlorine atom, a bromine atom or a methyl group,
$R^2$ denotes a $-CH_2-COOH$ group or a $-CH_2-COOR^6$ group,
$R^3$ denotes a hydrogen atom, a halogen atom, an alkyl group, an alkoxy group or a trifluoromethyl group,
$R^4$ has one of the meanings of $R^3$,
$R^5$ denotes a hydrogen atom, a halogen atom or an alkyl group,
$R^6$ denotes an alkyl group with 1 to 5 carbon atoms which is optionally substituted by hydroxyl, hydroxyalkoxy or alkanoyloxy groups, or a benzyl group and
n denotes 1 or 2,

and salts of the acids.

2. Phenylaminothiophenacetic acids of the general formula I*

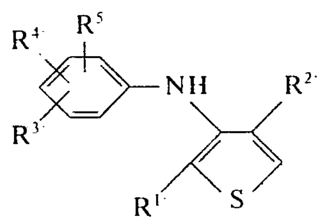

(I*)

wherein

$R^{1*}$ denotes a hydrogen atom, a chlorine atom, a bromine atom or a methyl group,
$R^{2*}$ denotes a $-CH_2-COOH$ group or a $-CH_2-COOR^{6*}$ group,
$R^{3*}$ denotes a hydrogen atom, a chlorine atom, a methyl group or a trifluoromethyl group,
$R^{4*}$ has one of the meanings of $R^{3*}$,
$R^{5*}$ denotes a hydrogen atom or a chlorine atom and
$R^{6*}$ denotes an alkyl group with 1 to 4 carbon atoms which is optionally substituted by an acetoxy group of a 2-hydroxyethoxy group, or a benzyl group,

and salts of the acids.

3. Compounds according to Claim 2, in which $R^{1*}$ denotes a hydrogen atom or a chlorine atom, $R^{2*}$ denotes a $-CH_2-COOH$ group or a $-CH_2-COOR^{6*}$ group, $R^{3*}$ denotes a chlorine atom, a methyl group or a trifluoromethyl group, $R^{4*}$ denotes a chlorine atom or a methyl group, $R^{5*}$ denotes a hydrogen atom or a chlorine atom and $R^{6*}$ denotes a 2-(2-hydroxyethoxy)-ethyl group, and the pharmacologically tolerated salts of the acids.

4. Compounds according to Claim 2, in which $R^{1*}$ denotes a hydrogen atom $R^{2*}$ denotes a $-CH_2-COOH$ group, $R^{3*}$ denotes a chlorine atom or a methyl group, $R^{4*}$ denotes a chlorine atom and $R^{5*}$ denotes a hydrogen atom, and their pharmacologically tolerated salts.

5. Phenylaminothiophenacetic acids of the general formula I**

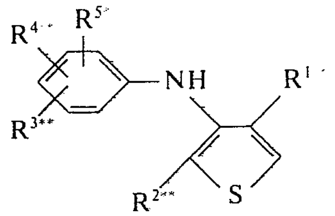

(I**)

wherein

$R^{1**}$ denotes a hydrogen atom or a methyl group,
$R^{2**}$ denotes a $-CH_2-COOH$ group or a $-CH_2-COOR^{6**}$ group,
$R^{3**}$ denotes a hydrogen atom, a chlorine atom, a methyl group or a trifluoromethyl group,
$R^{4**}$ has one of the meanings of $R^{3**}$,
$R^{5**}$ denotes a hydrogen atom or a chlorine atom and
$R^{6**}$ denotes an alkyl group with 1 to 4 carbon atoms which is optionally substituted by an acetoxy group or a 2-hydroxyethoxy group, or a benzyl group and salts of the acids.

6. Compounds according to Claim 5, in which $R^{1**}$ denotes a hydrogen atom, $R^{2**}$ denotes a $-CH_2-COOH$ group or a $-CH_2-COOR^{6**}$ group, $R^{3**}$ denotes a chlorine atom, a methyl group or a trifluoromethyl group, $R^{4**}$ denotes a chlorine atom or a methyl group; $R^{5**}$ denotes a hydrogen atom or chlorine atom and $R^{6**}$ denotes a 2-(2-hydroxyethoxy)-ethyl group, and the pharmacologically tolerated salts of the acids.

7. Compounds according to Claim 5, in which $R^{1**}$ denotes a hydrogen atom, $R^{2**}$ denotes a $-CH_2-COOH$ group, $R^{3**}$ denotes a chlorine atom or a methyl group, $R^{4**}$ denotes a chlorine atom and $R^{5**}$ denotes a hydrogen atom, and their pharmacologically tolerated salts.

8. 4-(2,6-Dichloro-anilino)-3-thiophenacetic acid and its pharmacologically tolerated salts.

9. Medicaments containing one or more compounds according to Claims 1 to 8.

10. Process for the preparation of phenylaminothiophenacetic acids according to Claim 1 and salts of the acids, characterised in that functional carboxylic acid derivatives of the general formula II

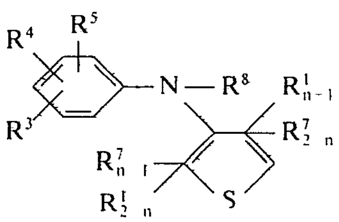

(II)

wherein $R^1$, $R^3$, $R^4$, $R^5$ and n have the meanings indicated in Claim 1, $R^7$ represents a $-CH_2-G$ group or, together with $R^8$, a $-CH_2-CO-$ group, $R^8$ represents a hydrogen atom or, together with $R^7$, a $-CO-CH_2-$ group and G represents a functional derivative of a carboxylic acid group, are lyolysed and the resulting acids or esters of the general formula I are then optionally converted into one another and/or into salts in the customary manner.

11. Process for the preparation of phenylaminothiophenacetic acids according to Claim 2 and salts of the acids, characterised in that functional phenylaminothiophenacetic acid derivatives of the general formula II*

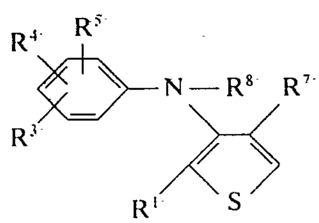

(II*)

wherein $R^{1*}$, $R^{3*}$, $R^{4*}$ and $R^{5*}$ have the meaning indicated in Claim 2, $R^{7*}$ represents a $-CH_2-G^*$ group or, together with $R^{8*}$, a $-CH_2-CO-$ group, $R^{8*}$ represents a hydrogen atom or, together with $R^{7*}$, a $-CO-CH_2-$ group and $G^*$ represents a functional derivative of a carboxylic acid group, are lyolysed and the resulting acids or esters of the general formula I* are then optionally halogenated and/or, in the customary manner, converted into one another and/or into salts.

12. Process for the preparation of phenylaminothiophenacetic acids according to Claim 5 and salts of the acids, characterised in that

a) functional phenylaminothiophenacetic acid derivatives of the general formula II**

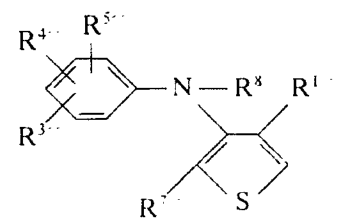

(II**)

wherein $R^{1**}$, $R^{3**}$, $R^{4**}$ and $R^{5**}$ have the meanings indicated in Claim 5, $R^{7**}$ represents a $-CH_2-G^{**}$ group or, together with $R^{8**}$, a $-CH_2-CO-$ group, $R^{8**}$ represents a hydrogen atom or, together with $R^{7**}$, a $-CO-CH_2-$ group and $G^{**}$ represents a functional derivative of a carboxylic acid group, are lyolysed and the resulting acids or esters of the general formula I** are then optionally converted into one another and/or into salts in the customary manner, or

b) a phenylaminothiophenacetic acid derivative of the general formula III**

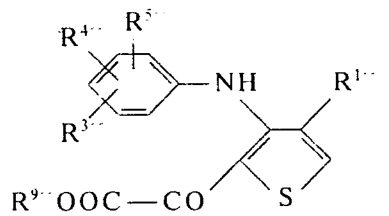

(III**)

wherein $R^{1**}$, $R^{3**}$, $R^{4**}$ and $R^{5**}$ have the meanings indicated in Claim 5 and $R^{9**}$ represents a hydrogen atom or an alkyl group with 1 to 5 carbon atoms, or a salt thereof, is reduced and the resulting acids or esters of the general formula I** are then optionally converted into one another and/or into salts in the customary manner.

13. Active compounds according to Claims 1 to 8 for use in combating illnesses connected with inflammation, pain or fever.

14. Use of active compounds according to Claims 1 to 8 for the preparation of medicaments which are employed for combating illnesses connected with inflammation, pain or fever.

**Claims for the contracting State: AT**

1. Process for the preparation of phenylaminothiophenacetic acids of the general formula I

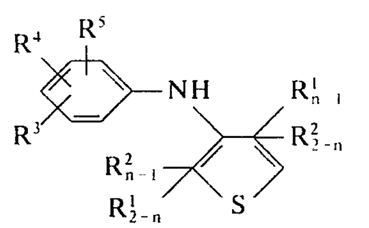

(I)

wherein

$R^1$ denotes a hydrogen atom, a chlorine atom, a bromine atom or a methyl group,
$R^2$ denotes a $-CH_2-COOH$ group or a $-CH_2-COOR^6$ group,
$R^3$ denotes a hydrogen atom, a halogen atom, an alkyl group, an alkoxy group or a trifluoromethyl group,
$R^4$ has one of the meanings of $R^3$,
$R^5$ denotes a hydrogen atom, a halogen atom or an alkyl group,
$R^6$ denotes an alkyl group with 1 to 5 carbon atoms which is optionally substituted by hydroxyl, hydroxyalkoxy or alkanoyloxy groups, or a benzyl group and
n denotes 1 or 2,

and salts of the acids, characterised in that functional carboxylic acid derivatives of the general formula II

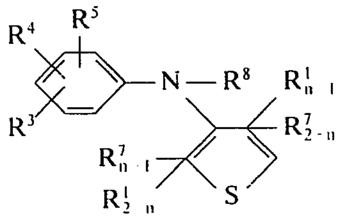

(II)

wherein $R^1$, $R^3$, $R^4$, $R^5$ and n have the meanings indicated above, $R^7$ represents a $-CH_2-G$ group or, together with $R^8$, a $-CH_2-CO-$ group, $R^8$ represents a hydrogen atom or, together with $R^7$, a $-CO-CH_2-$ group and G represents a functional derivative of a carboxylic acid group, are lyolysed and the resulting acids or esters of the general formula I are then optionally converted into one another and/or into salts in the customary manner.

2. Process for the preparation of phenylaminothiophenacetic acids of the general formula I*

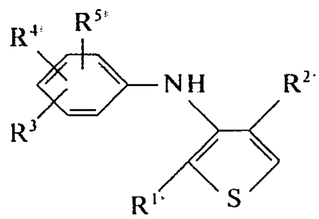

(I*)

wherein

$R^{1*}$ denotes a hydrogen atom, a chlorine atom, a bromine atom or a methyl group,
$R^{2*}$ denotes a $-CH_2-COOH$ group or a $-CH_2-COOR^{6*}$ group,
$R^{3*}$ denotes a hydrogen atom, a chlorine atom, a methyl group or a trifluoromethyl group,
$R^{4*}$ has one of the meanings of $R^{3*}$,
$R^{5*}$ denotes a hydrogen atom or a chlorine atom and
$R^{6*}$ denotes an alkyl group with 1 to 4 carbon atoms which is optionally substituted by an acetoxy group or a 2-hydroxyethoxy group, or a benzyl group,

and salts of the acids, characterised in that functional phenylaminothiophenacetic acid derivatives of the general formula II*

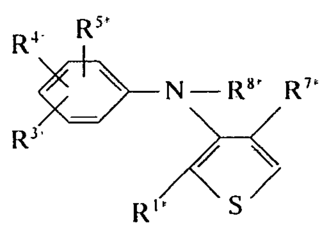

(II*)

wherein $R^{1*}$, $R^{3*}$, $R^{4*}$ and $R^{5*}$ have the meanings indicated above, $R^{7*}$ represents a $-CH_2-G^*$ group or, together with $R^{8*}$, a $-CH_2-CO-$ group, $R^{8*}$ represents a hydrogen atom or, together with $R^{7*}$, a $-CO-CH_2-$ group and G* represents a functional derivative of a carboxylic acid group, are lyolysed and the resulting acids or esters of the general formula I* are then optionally halogenated and/or, in the customary manner, converted into one another and/or into salts.

3. Process for the preparation of phenylaminothiophenacetic acids of the general formula I**

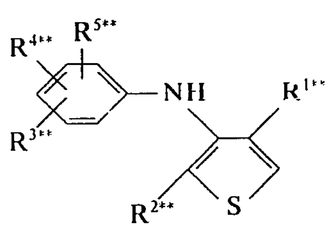

(I**)

wherein

$R^{1**}$ denotes a hydrogen atom or a methyl group,
$R^{2**}$ denotes a $-CH_2-COOH$ group or a $-CH_2-COOR^{6**}$ group,
$R^{3**}$ denotes a hydrogen atom, a chlorine atom, a methyl group or a trifluoromethyl group,
$R^{4**}$ has one of the meanings of $R^{3**}$

$R^{5**}$ denotes a hydrogen atom or a chlorine atom and
$R^{6**}$ denotes an alkyl group with 1 to 4 carbon atoms which is optionally substituted by an acetoxy group or a 2-hydroxyethoxy group, or a benzyl group,

and salts of the acids, characterised in that

a) functional phenylaminothiophenacetic acid derivatives of the general formula II**

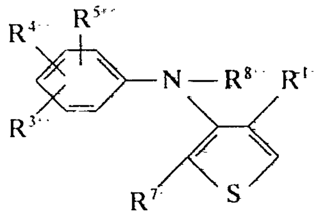

(II**)

wherein $R^{1**}$, $R^{3**}$, $R^{4**}$ and $R^{5**}$ have the meanings indicated above, $R^{7**}$ represents a $-CH_2-G^{**}$ group or, together with $R^{8**}$, a $-CH_2-CO-$ group, $R^{8**}$ represents a hydrogen atom or, together with $R^{7**}$, a $-CO-CH_2-$ group and $G^{**}$ represents a functional derivative of a carboxylic acid group, are lyolysed and the resulting acids or esters of the general formula I** are then optionally converted into one another and/or into salts in the customary manner, or

b) a phenylaminothiophenacetic acid derivative of the general formula III**

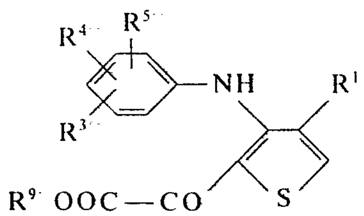

(III**)

wherein $R^{1**}$, $R^{3**}$, $R^{4**}$ and $R^{5**}$ have the meanings indicated above and $R^{9**}$ represents a hydrogen atom or an alkyl group with 1 to 5 carbon atoms, or a salt thereof, is reduced and the resulting acids or esters of the general formula I** are then optionally converted into one another and/or into salts in the customary manner.

4. Process according to Claims 1 or 2 or 3, characterized in that the functional carboxylic acid derivatives G and G* and G**, respectively, represent a nitrile group, a carboxylic acid alkyl ester group with 1 to 7 carbon atoms in the alkyl radical, a carboxylic acid benzyl ester group or a carboxylic acid amide group.

5. Process according to Claims 1 or 2 or 3, characterized in that the lyolysis is carried out in the form of solvolysis.

6. Process according to Claim 2, characterized in that compounds of the general formula I* wherein $R^{1*}$ denotes a hydrogen atom or a chlorine atom, $R^{2*}$ denotes a $-CH_2-COOH$ group or a $-CH_2-COOR^{6*}$ group, $R^{3*}$ denotes a chlorine atom, a methyl group or a trifluoromethyl group, $R^{4*}$ denotes a chlorine atom or a methyl group, $R^{5*}$ denotes a hydrogen atom or a chlorine atom and $R^{6*}$ denotes a 2-(2-hydroxyethoxy)-ethyl group, or the pharmacologically tolerated salts of the acids are prepared.

7. Process according to Claim 2, characterized in that compounds of the general formula I* wherein $R^{1*}$ denotes a hydrogen atom, $R^{2*}$ denotes a $-CH_2-COOH$ group, $R^{3*}$ denotes a chlorine atom or a methyl group, $R^{4*}$ denotes a chlorine atom and $R^{5*}$ denotes a hydrogen atom, or the pharmacologically tolerated salts of the acids are prepared.

8. Process according to Claim 2, characterized in that 4-(2,6-dichloroanilino)-3-thiophenacetic acid or its pharmacologically tolerated salts are prepared by hydrolysis of 4-(2,6-dichloro-anilino)-3-thiophenacetonitrile or 4-(2,6-dichloro-anilino)-3-thiophenacetic acid alkyl esters and optionally subsequent conversion into the salts.

9. Process according to Claim 3, characterized in that compounds of the general formula I** wherein $R^{1**}$ denotes a hydrogen atom, $R^{2**}$ denotes a $-CH_2-COOH$ group or a $-CH_2-COOR^{6**}$ group, $R^{3**}$ denotes a chlorine atom, a methyl group or a trifluoromethyl group, $R^{4**}$ denotes a chlorine atom or a methyl group, $R^{5**}$ denotes a hydrogen atom or a chlorine atom, and $R^{6**}$ denotes a 2-(2-hydroxy-ethoxy) ethyl group, or the pharmacologically tolerated salts of the acids are prepared.

10. Process according to Claim 3, characterized in that compounds of the general formula I** wherein $R^{1**}$ denotes a hydrogen atom, $R^{2**}$ denotes a $-CH_2-COOH$ group, $R^{3**}$ denotes a chlorine atom or a methyl group, $R^{4**}$ denotes a chlorine atom and $R^{5**}$ denotes a hydrogen atom, or their pharmacologically tolerated salts are prepared.

**Revendications pour les Etats contractants: CH, DE, IT, LU, NL, SE**

1. Acides phénylaminothiophène-acétiques de la formule générale I

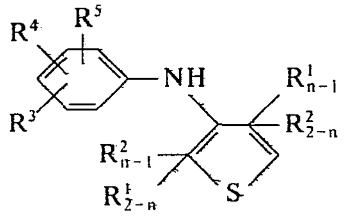

(I)

dans laquelle

$R^1$ représente un atome d'hydrogène, un atome de chlore, un atome de brome ou un groupe méthyle,
$R^2$ représente un groupe $-CH_2-COOH$ ou un groupe $-CH_2-COOR^6$, et
$R^3$ représente un atome d'hydrogène, un atome d'halogène, un groupe alcoyle, un groupe alcoxy- ou un groupe trifluorométhyle,
$R^4$ a l'une des significations de $R^3$,
$R^5$ représente un atome d'hydrogène, un atome d'halogène ou un groupe alcoyle,
$R^6$ représente un groupe alcoyle comportant de 1à 5 atomes de carbone, éventuellement substitué par des groupes hydroxy-, hydroxyalcoxy- ou alcanoyloxy-, ou un groupe benzyle, et
n représente 1 ou 2,

ainsi que les sels des acides.

2. Acides phénylaminothiophène-acétiques de la formule générale I*

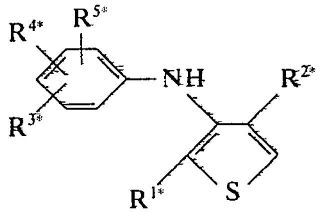

(I*)

dans laquelle

$R^{1*}$ représente un atome d'hydrogène, un atome de chlore, un atome de brome ou un groupe méthyle,
$R^{2*}$ représente un groupe $-CH_2-COOH$ ou un groupe $-CH_2-COOR^{6*}$ et
$R^{3*}$ représente un atome d'hydrogène, un atome de chlore, un groupe, méthyle ou un groupe trifluorométhyle,
$R^{4*}$ a l'une des significations de $R^{3*}$,
$R^{5*}$ représente un atome d'hydrogéne ou un atome de chlore et
$R^{6*}$ représente un groupe alcoyle comportant de 1 à 4 atomes de carbone, éventuellement substitué par un groupe acêtoxy- ou un groupe 2-hydroxyéthoxy-, ou un groupe benzyle,

ainsi que les sels des acides.

3. Composés selon la Revendication 2, dans laquelle $R^{1*}$ représente un atome d'hydrogène ou un atome de chlore, $R^{2*}$ représente un groupe $-CH_2-COOH$ ou un groupe $-CH_2-COOR^{6*}$, $R^{3*}$ représente un atome de chlore, un groupe méthyle ou un groupe trifluorométhyle, $R^{4*}$ représente un atome de chlore ou un groupe méthyle, $R^{5*}$ représente un atome d'hydrogene ou un atome de chlore et $R^{6*}$ représente un groupe 2-(2-hydroxyéthoxy)-éthyle, ainsi que les sels pharmacologiquement compatibles des acides.

4. Composés selon la Revendication 2, dans laquelle $R^{1*}$ représente un atome d'hydrogène, $R^{2*}$ représente un groupe $-CH_2-COOH$, $R^{3*}$ représente un atome de chlore ou un groupe méthyle, $R^{4*}$ représente un atome de chlore et $R^{5*}$ représente un atome d'hydrogène, ainsi que leurs sels pharmacologiquement compatibles.

5. Acides phénylaminothiophène-acétiques de la formule générale I**

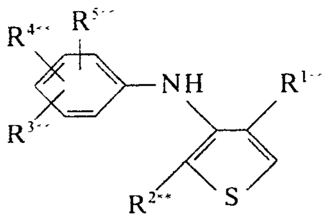

(I**)

dans laquelle

$R^{1**}$ représente un atome d'hydrogène ou un groupe méthyle,
$R^{2**}$ représente un groupe $-CH_2-COOH$ ou un groupe $-CH_2-COOR^{6**}$ et
$R^{3**}$ représente un atome d'hydrogène, un atome de chlore, un groupe méthyle ou un groupe trifluorométhyle,
$R^{4**}$ a l'une des significations de $R^{3**}$,
$R^{5**}$ représente un atome d'hydrogène ou un atome de chlore et
$R^{6**}$ représente un groupe alcoyle comportant de 1 à 4 atomes de carbone, éventuellement substitué par un groupe acétoxy- ou une groupe 2-hydroxyéthoxy-, ou un groupe benzyle,

ainsi que les sels des acides.

6. Composés selon la Revendication 5, dans laquelle $R^{1**}$ représente un atome d'hydrogène, $R^{2**}$ représente un groupe $-CH_2-COOH$ ou un groupe $-CH_2-COOR^{6**}$, $R^{3**}$ représente un atome de chlore, un groupe méthyle ou un groupe trifluorométhyle, $R^{4**}$ représente un atome de chlore ou un groupe méthyle, $R^{5**}$ représente un atome d'hydrogène ou un atome de chlore et $R^{6**}$ représente un groupe 2-(2-hydroxyéthoxy)-éthyle, ainsi que les sels pharmacologiquement compatibles des acides.

7. Composés selon la Revendication 5, dans laquelle $R^{1**}$ représente un atome d'hydrogène, $R^{2**}$ représente un groupe $-CH_2-COOH$, $R^{3**}$ représente un atome de chlore ou un groupe méthyle, $R^{4**}$ représente un atome de chlore et $R^{5**}$ représente un atome d'hydrogène, ainsi que leurs sels pharmacologiquement compatibles.

8. L'acide 4-(2,6-dichloroanilino)-3-thiophène-acétique et ses sels pharmacologiquement compatibles.

9. Médicaments caractérisés en ce qu'ils contiennent un ou plusieurs composés selon les Revendications 1 à 8.

10. Procédé de préparation d'acides phénylaminothiophène-acétiques selon la Revendication 1 et les sels des acides, caractérisé en ce qu'on lyolyse des dérivés fonctionnels d'acides carboxyliques, qui répondent à la formule générale II

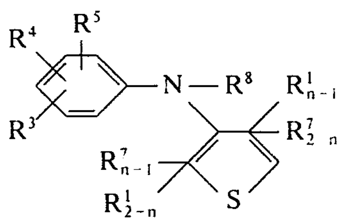

(II)

dans laquelle $R^1$, $R^3$, $R^4$, $R^5$ et n ont les significations indiquées dans la Revendication 1, $R^7$ représente un groupe $-CH_2-G$ ou, conjointement avec $R^8$, un groupe $-CH_2-CO-$, $R_8$ représente un atome d'hydrogène ou, conjointement avec $R^7$, un groupe $-CO-CH_2-$ et G représente un dérivé fonctionnel d'un groupe acide carboxylique, après quoi l'on transforme éventuellement de façon usuelle, les acides ou les esters de formule générale I obtenus, les uns dans les autres ou en leurs sels.

11. Procédé de préparation d'acides phénylaminothiophène-acétiques selon la Revendication 2 et les sels des acides, caractérisé en ce qu'on lyolyse des dérivés fonctionnels d'acides phénylaminothiophène-acétiques qui répondent à la formule gènerale II*

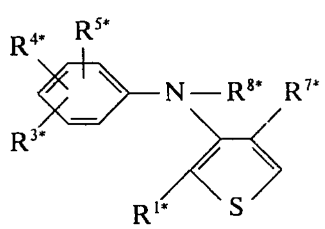

(II*)

dans laquelle $R^{1*}$, $R^{3*}$, $R^{4*}$ et $R^{5*}$ ont les significations indiquées dans la Revendication 2, $R^{7*}$ représente un groupe $-CH_2-G^*$ ou, conjointement avec $R^{8*}$, un groupe $-CH_2-CO-$, $R^{8*}$

représente un atome d'hydrogène ou, conjointement avec $R^{7*}$, un groupe $-CO-CH_2-$ et $G^*$ représente un dérivé fonctionnel d'un groupe acide carboxylique, après quoi l'on halogène éventuellement les acides ou les esters de formule générale I* obtenus et/ou les transforme de façon usuelle, les uns dans les autres et/ou en leurs sels.

12. Procédé de préparation d'acides phénylaminothiophène-acétiques selon la Revendication 5, caractérisé en ce

a) qu'on lyolyse des dérivés fonctionnels d'acides phénylaminothiophène-acétiques qui répondent à la formule générale II**

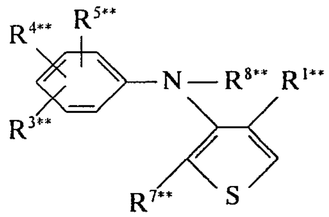

(II**)

dans laquelle $R^{1**}$, $R^{3**}$, $R^{4**}$ et $R^{5**}$ ont les significations indiquées dans la Revendication 5, $R^{7**}$ représente un groupe $-CH_2-G^{**}$ ou, conjointement avec $R^{8**}$, un groupe $-CH_2-CO-$, $R^{8**}$ représente un atome d'hydrogène ou, conjointement avec $R^{7**}$, un groupe $-CO-CH_2-$ et $G^{**}$ représente un dérivé fonctionnel d'un groupe acide carboxylique, après quoi l'on transforme éventuellement de façon usuelle, les acides ou les esters de formule générale I** obtenus, les uns dans les autres et/ou en leurs sels, ou

b) que l'on réduit un dérivé d'acide phénylaminothiophène-acétique répondant à la formule générale III**

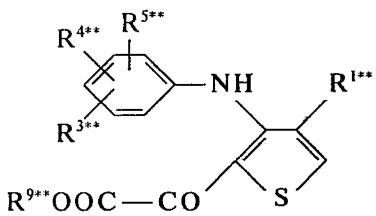

(III**)

dans laquelle $R^{1**}$, $R^{3**}$, $R^{4**}$ et $R^{5**}$ ont les significations indiquées dans la Revendication 5 et $R^{9**}$ représente un atome d'hydrogène ou un groupe alcoyle comportant de 1 à 5 atomes de carbone, ou un sel de celui-ci, après quoi l'on transforme éventuellement de façon usuelle, les acides ou les esters de formule générale I** obtenus, les uns dans les autres et/ou en leurs sels.

13. Composés actifs selon les Revendications 1 à 8 pour utilisation pour traitement des maladies, dans lesquelles apparaissent les symptomes d'inflammations, de douleurs ou de fièvre.

14. Utilisation des composés actifs selon les Revendications 1 à 8 pour la preparation de médicaments qui sont mis en oeuvre pour lutter contre les maladies, dans lesquelles apparaissent les symptomes d'inflammations, de douleurs ou de fièvre.

**Revendications pour l'Etat contractant: AT**

1. Procédé de préparation d'acides phénylaminothiophène-acétiques de la formule générale I

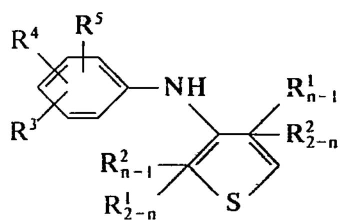

(I)

dans laquelle

$R^1$ représente un atome d'hydrogène, un atome de chlore, un atome de brome ou un groupe méthyle,

$R^2$ représente un groupe $-CH_2-COOH$ ou un groupe $-CH_2-COOR^6$, et

$R^3$ représente un atome d'hydrogène, un atome d'halogène, un groupe alcoyle, un groupe alcoxy- ou un groupe trifluorométhyle,

$R^4$ a l'une des significations de $R^3$,
$R^5$ représente un atome d'hydrogene, un atome d'halogène ou un groupe alcoyle,
$R^6$ représente un groupe alcoyle comportant de 1 à 5 atomes de carbone, éventuellement substitué par des groupes hydroxy-, hydroxyalcoxy- ou alcanoyloxy-, ou un groupe benzyle, et
n représente 1 ou 2,

ainsi que les sels des acides, caractérisé en ce qu'on lyolyse des dérivés fonctionnels d'acides carboxyliques, qui répondent à la formule générale II

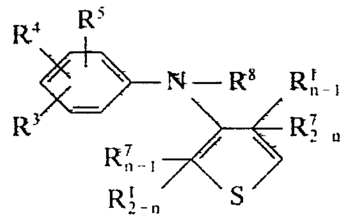

(II)

dans laquelle $R^1$, $R^3$, $R^4$, $R^5$ et n ont les significations indiquées précédemment, $R^7$ représente un groupe $-CH_2-G$ ou, conjointement avec $R^8$, un groupe $-CH_2-CO-$, $R^8$ représente un atome d'hydrogène ou, conjointement avec $R^7$, un groupe $-CO-CH_2-$ et G représente un dérivé fonctionnel d'un groupe acide carboxylique, après quoi l'on transforme éventuellement de façon usuelle, les acides ou les esters de formule générale I obtenus, les uns dans les autres ou en leurs sels.

2. Procédé de préparation d'acides phénylaminothiophène-acétiques de la formule générale I*

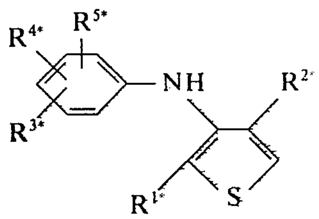

(I*)

dans laquelle

$R^{1*}$ représente un atome d'hydrogène, un atome de chlore, un atome de brome ou un groupe méthyle,
$R^{2*}$ représente un groupe $-CH_2-COOH$ ou un groupe $-CH_2-COOR^{6*}$ et
$R^{3*}$ représente un atome d'hydrogène, un atome de chlore, un groupe méthyle ou un groupe trifluorométhyle,
$R^{4*}$ a l'une des significations de $R^{3*}$,
$R^{5*}$ représente un atome d'hydrogène ou un atome de chlore et
$R^{6*}$ représente un groupe alcoyle comportant de 1 à 4 atomes de carbone, éventuellement substitué par un groupe acétoxy- ou un groupe 2-hydroxyéthoxy-, ou un groupe benzyle,

ainsi que les sels des acides, caractérisé en ce qu'on lyolyse des dérivés fonctionnels d'acides phénylaminothiophène-acétiques qui répondent à la formule générale II*

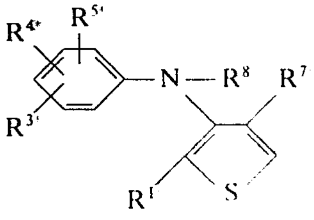

(II*)

dans laquelle $R^{1*}$, $R^{3*}$, $R^{4*}$ et $R^{5*}$ ont les significations indiquées précédemment, $R^{7*}$ représenté un groupe $-CH_2-G^*$ ou, conjointement avec $R^{8*}$, un groupe $-CH_2-CO-$, $R^{8*}$ représente un atome d'hydrogène ou, conjointement avec $R^{7*}$, un groupe $-CO-CH_2-$ et $G^*$ représente un dérivé fonctionnel d'un groupe acide carboxylique, après quoi l'on halogène éventuellement les acides ou les esters de formule générale I* obtenus et/ou on les transforme de façon usuelle, les uns dans les autres et/ou en leurs sels.

3. Procédé de préparation d acides phénylaminothiophène-acétique de la formule générale I**

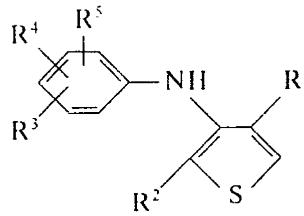

(I**)

dans laquelle

$R^{1**}$ représente un atome d'hydrogène ou un groupe méthyle,
$R^{2**}$ représente un groupe $-CH_2-COOH$ ou un groupe $-CH_2-COOR^{6**}$ et
$R^{3**}$ représente un atome d'hydrogène, un atome de chlore, un groupe méthyle ou un groupe trifluorométhyle,
$R^{4**}$ a l'une des significations de $R^{3**}$,
$R^{5**}$ représente un atome d'hydrogène ou un atome de chlore et
$R^{6**}$ représente un groupe alcoyle comportant de 1 à 4 atomes de carbone, éventuellement substitué par un groupe acétoxy- ou une groupe 2-hydroxyéthoxy-, ou un groupe benzyle,

ainsi que les sels des acides, caractérisé en ce
a) qu'on lyolyse des dérivés fonctionnels d'acides phénylaminothiophène-acétiques qui répondent à la formule générale II**

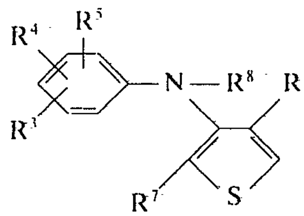

(II**)

dans laquelle $R^{1**}$, $R^{3**}$, $R^{4**}$ et $R^{5**}$ ont les significations indiquées précédemment, $R^{7**}$ représente un groupe $-CH_2-G^{**}$ ou, conjointement avec $R^{8**}$, un groupe $-CH_2-CO-$, $R^{8**}$ représente un atome d'hydrogène ou, conjointement avec $R^{7**}$, un groupe $-CO-CH_2-$ et $G^{**}$ représente un dérivé fonctionnel d'un groupe acide carboxylique, après quoi l'on transforme éventuellement de façon usuelle, les acides ou les esters de formule générale I** obtenus, les uns dans les autres et/ou en leurs sels, ou
b) que l'on réduit un dérivé d'acide phénylaminothiophène-acétique répondant à la formule générale III**

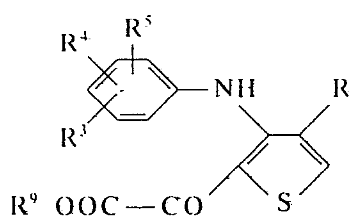

(III**)

dans laquelle $R^{1**}$, $R^{3**}$, $R^{4**}$ et $R^{5**}$ ont les significations indiquées précédemment et $R^{9**}$ représente un atome d'hydrogène ou un groupe alcoyle comportant de 1 à 5 atomes de carbone, ou un sel de celui-ci, après quoi l'on transforme éventuellement de façon usuelle, les acides ou les esters de formule générale I** obtenus, les uns dans les autres et/ou en leurs sels.

4. Procédé selon les Revendications 1 ou 2 ou 3, caractérisé en ce que le dérivé fonctionnel du groupe acide carboxylique G ou G* ou G** représente un groupe nitrile, un groupe alcoylester d'acide carboxylique dont le reste alcoyle comporte de 1 à 7 atomes de carbone, un groupe benzylester d'acide carboxylique ou un group amide carboxylique.

5. Procédé selon les Revendications 1 ou 2 ou 3, caractérisé en ce qu'on effectue la lyolyse sous la forme de la solvolyse.

6. Procédé selon la Revendication 2, caractérisé en ce qu'on prépare les composés de la formule générale I*, dans laquelle $R^{1*}$ représente un atome d'hydrogène ou un atome de chlore, $R^{2*}$ représente un groupe $-CH_2-COOH$ ou un groupe $-CH_2-COOR^{6*}$, $R^{3*}$ représente un atome de chlore, un groupe méthyle ou un groupe trifluorométhyle, $R^{4*}$ représente un atome de chlore ou un groupe méthyle, $R^{5*}$ représente un atome d'hydrogène ou un atome de chlore et $R^{6*}$ représente un groupe 2-(2-hydroxyéthoxy) éthyle, ou les sels pharmacologiquement compatibles des acides

7. Procédé selon la Revendication 2, caractérisé en ce qu'on prépare les composés de la formule générale I*, dans laquelle R1* représente un atome d'hydrogène, R2* représente un groupe $-CH_2-COOH$, représente un atome de chlore ou un groupe méthyle, R4* représente un atome de chlore et R5* représente un atome d hydrogène, ou leurs sels pharmacologiquement compatibles.

8. Procédé selon la Revendication 2, caractérisé en ce qu'on prépare l'acide 4-(2,6-dichloroanilino)-3-thiophène-acétique ou sels pharmacologiquement compatibles par hydrolyse du 4-(2,6-dichloroanilino)-3-thiophène-acétonitrile ou des alcoylester de l'acide 4-(2,6-dichloroanilino)-3-thiophène-acetique et, en cas echeant, transformation suivante en les sels.

9. Procédé selon la Revendication 3, caractérisé en ce qu'on prépare les composés de la formule générale I**, dans laquelle R1** représente un atome d'hydrogène, R2** représente un groupe $-CH_2-COOH$ ou un groupe $-CH_2-COOR^{6**}$, R3** représente un atome de chlore, un groupe méthyle ou un groupe trifluorométhyle, R4** représente un atome de chlore ou un groupe méthyle, R5** représente un atome d'hydrogène ou un atome de chlore et R6** représente un groupe 2-(2-hydroxyéthoxy)-éthyle, ou les sels pharmacologiquement compatibles des acides.

10. Procédé selon la Revendication 3, caractérisé en ce qu'on prépare les composés de la formule générale I**, dans laquelle R1** représente un atome d'hydrogène, R2** représente un groupe $-CH_2-COOH$, R3** représente un atome de chlore ou un groupe méthyle, R4** représente un atome de chlore et R5** représente un atome d'hydrogène, ou ses sels pharmacologiquement compatibles.